# EUROPEAN PATENT APPLICATION

(11) **EP 2 147 973 A1**
(43) Date of publication of application: **27.01.2010**
(21) Application number: 08752842.8
(22) Date of filing: 09.05.2008
(51) Int. Cl.: C12N 15/00, C12N 1/15, C12N 1/19, C12N 1/21, C12N 5/10, C12N 15/09, C12P 7/44, C12P 17/04

(54) **GENE-DISRUPTED STRAIN, RECOMBINANT PLASMID, TRANSFORMANT AND METHOD OF PRODUCING 3-CARBOXYMUCONOLACTONE**

(30) Priority: 11.05.2007 JP 2007126990
(71) Applicant: KABUSHIKI KAISHA TOYOTA JIDOSHOKKI, Kariya-shi, Aichi 448-8671 (JP); National University Corporation Tokyo University of Agriculture and Technology, Fuchu-shi, Tokyo 183-8538 (JP); Nagaoka University of Technology, Nagaoka-shi Niigata 940-2188 (JP); FORESTRY AND FOREST PRODUCTS RESEARCH INSTITUTE, Tsukuba-shi Ibaragi 305-8687 (JP)
(72) Inventor: SHIMO, Toshihisa, Kariya-shi Aichi 448-8671 (JP); MASE, Kohei, Kariya-shi Aichi 448-8671 (JP); KATAYAMA, Yoshihiro, Fuchu-shi Tokyo 183-8538 (JP); MASAI, Eiji, Nagaoka-shi Niigata 940-2188 (JP); FUKUDA, Masao, Nagaoka-shi Niigata 940-2188 (JP); SHIGEHARA, Kiyotaka, Fuchu-shi Tokyo 183-8538 (JP); OHARA, Seiji, Tsukuba-shi Ibaraki 305-8687 (JP); NAKAMURA, Masaya, Tsukuba-shi Ibaraki 305-8687 (JP); OTSUKA, Yuichiro, Tsukuba-shi Ibaraki 305-8687 (JP)
(74) Representative: TBK-Patent
(86) International application number: PCT/JP2008/058988
(87) International publication number: WO 2008/143150

(57) **Abstract**

Industrial-scale fermentative production of 3-carboxy-cis,cis-muconic acid from terephthalic acid.
Also, a protocatechuate 4,5-ring-cleaving enzyme gene-disrupted strain in which the gene coding for (a) the amino acid sequence set forth in SEQ ID NO: 1 or 3, or (b) the amino acid sequence set forth in SEQ ID NO: 1 or 3 which has a deletion, substitution, addition and/or insertion of one or more amino acids and exhibits protocatechuate 4,5-ring cleavage activity, present in the chromosomal DNA of microbial cells, has been disrupted; recombinant plasmids comprising the Tph gene and protocatechuate 3,4-dioxygenase gene; transformants obtained by introducing the recombinant plasmids into the disrupted strain; and a process for production of 3-carboxy-cis,cis-muconic acid and/or 3-carboxymuconolactone **characterized by** culturing the transformants in the presence of terephthalic acid.

SEQUENCE LISTING

<110> TOYOTA INDUSTRIES CORPORATION/ Tokyo University of
Agriculture and Technology/ Nagaoka University of
Technology/ Forestry and Forest Products Research
Institute

<120> Mutant, Recombinat Plasmid, Transformant and Method for
Producing 3-Carboxymuconolactone

<130> V576-PCT

<140> PCT/JP 2008/058988
<141> 2008-05-09

<150> JP 2007-126990
<151> 2007-05-11

<160> 16

<170> PatentIn version 3.4

<210> 1
<211> 814
<212> PRT
<213> Comamonas sp.E6

<400> 1

Met Glu Thr Ala Leu Ala Ala Arg Gly Ile Leu Glu Thr His Arg Ala
1               5                   10                  15

Leu Ala Ser Glu Arg Val Ala Leu Pro His Glu Thr His Arg Ser Glu
            20                  25                  30

Arg His Ile Ser Val Ala Leu Pro Arg Ala Leu Ala Ile Leu Glu Gly
        35                  40                  45

Leu Tyr Ala Leu Ala Ala Leu Ala Met Glu Thr Ala Ser Pro Met Glu
    50                  55                  60

Thr Gly Leu Tyr Leu Tyr Ser Thr His Arg Gly Leu Asn Gly Leu Ala
65                  70                  75                  80

Leu Ala Thr Tyr Arg Thr Arg Pro Ala Leu Ala Pro Arg Leu Glu Pro
                85                  90                  95

His Glu Leu Tyr Ser Gly Leu Tyr Thr Tyr Arg Ala Ser Pro Pro His
            100                 105                 110

Glu Ser Glu Arg Ala Arg Gly Gly Leu Asn Thr Arg Pro Met Glu Thr
        115                 120                 125

Leu Tyr Ser Ala Ser Pro Ala Ser Asn Leu Tyr Ser Pro Arg Ala Ser
    130                 135                 140

Pro Val Ala Leu Ile Leu Glu Pro His Glu Leu Glu Val Ala Leu Thr
145                 150                 155                 160

Tyr Arg Ala Ser Asn Ala Ser Pro His Ile Ser Ala Leu Ala Thr His
                165                 170                 175

Arg Ala Leu Ala Pro His Glu Ser Glu Arg Leu Glu Ala Ser Pro Cys
            180                 185                 190

Tyr Ser Ile Leu Glu Pro Arg Thr His Arg Pro His Glu Ala Leu Ala
        195                 200                 205

Ile Leu Glu Gly Leu Tyr Thr His Arg Ala Leu Ala Ala Leu Ala Gly
    210                 215                 220

Leu Pro His Glu Gly Leu Asn Pro Arg Ala Leu Ala Ala Ser Pro Gly
225                 230                 235                 240

Leu Gly Leu Tyr Thr Arg Pro Gly Leu Tyr Pro Arg Ala Arg Gly Pro
                245                 250                 255

Arg Val Ala Leu Pro Arg Leu Tyr Ser Val Ala Leu Val Ala Leu Gly
            260                 265                 270

Leu Tyr His Ile Ser Pro Arg Ala Ser Pro Leu Glu Ala Leu Ala Ser
        275                 280                 285

Glu Arg His Ile Ser Ile Leu Glu Ala Leu Ala Gly Leu Asn Ser Glu
    290                 295                 300

Arg Val Ala Leu Ile Leu Glu Gly Leu Asn Gly Leu Asn Ala Ser Pro
305                 310                 315                 320

Pro His Glu Ala Ser Pro Leu Glu Thr His Arg Ile Leu Glu Val Ala
                325                 330                 335

Leu Ala Ser Asn Leu Tyr Ser Met Glu Thr Ala Ser Pro Val Ala Leu
            340                 345                 350

Ala Ser Pro His Ile Ser Gly Leu Tyr Leu Glu Thr His Arg Val Ala
        355                 360                 365

Leu Pro Arg Leu Glu Ser Glu Arg Leu Glu Met Glu Thr Cys Tyr Ser
    370                 375                 380

Gly Leu Tyr Gly Leu Gly Leu Asn Ala Ser Pro Pro Arg Leu Tyr Ser
385                 390                 395                 400

Thr His Arg Gly Leu Tyr Ser Glu Arg Thr Arg Pro Pro Arg Cys Tyr
                405                 410                 415

Ser Pro Arg Val Ala Leu Ile Leu Glu Pro Arg Pro His Glu Ala Leu
            420                 425                 430

Ala Val Ala Leu Ala Ser Asn Val Ala Leu Val Ala Leu Gly Leu Asn
        435                 440                 445

Thr Tyr Arg Pro Arg Val Ala Leu Pro Arg Thr His Arg Gly Leu Tyr
    450                 455                 460

Gly Leu Asn Ala Arg Gly Cys Tyr Ser Pro His Glu Ala Ser Asn Leu
465                 470                 475                 480

Glu Gly Leu Tyr Ala Arg Gly Ala Leu Ala Ile Leu Glu Ala Arg Gly
                485                 490                 495

Leu Tyr Ser Ala Leu Ala Val Ala Leu Gly Leu Ser Glu Arg Thr Tyr
            500                 505                 510

Arg Ala Ser Pro Gly Leu Asn Ala Ser Pro Ile Leu Glu Ala Ser Asn
        515                 520                 525

Val Ala Leu His Ile Ser Ile Leu Glu Thr Arg Pro Gly Leu Tyr Thr
    530                 535                 540

His Arg Gly Leu Tyr Gly Leu Tyr Met Glu Thr Ser Glu Arg His Ile
545                 550                 555                 560

Ser Gly Leu Asn Leu Glu Gly Leu Asn Gly Leu Tyr Ala Leu Ala Ala
                565                 570                 575

Arg Gly Ala Leu Ala Gly Leu Tyr Leu Glu Ile Leu Glu Ala Ser Asn
            580                 585                 590

Leu Tyr Ser Gly Leu Thr Arg Pro Ala Ser Pro Ala Ser Asn Gly Leu
        595                 600                 605

Asn Pro His Glu Leu Glu Ala Ser Pro Leu Glu Leu Glu Ile Leu Glu
    610                 615                 620

Gly Leu Ala Ser Asn Pro Arg His Ile Ser Gly Leu Tyr Leu Glu Ala
625                 630                 635                 640

Leu Ala Gly Leu Asn Met Glu Thr Pro Arg His Ile Ser Ile Leu Glu
                645                 650                 655

Ala Ser Pro Thr Tyr Arg Val Ala Leu Ala Arg Gly Gly Leu Ala Leu
            660                 665                 670

Ala Gly Leu Tyr Ser Glu Arg Gly Leu Gly Leu Tyr Ile Leu Glu Gly
        675                 680                 685

Leu Leu Glu Val Ala Leu Met Glu Thr Thr Arg Pro Leu Glu Ile Leu
    690                 695                 700

Glu Ala Leu Ala Ala Arg Gly Gly Leu Tyr Ala Leu Ala Met Glu Thr
705                 710                 715                 720

Ser Glu Arg Ala Ser Pro Val Ala Leu Ala Ser Pro Gly Leu Tyr Pro
                725                 730                 735

Arg Ala Leu Ala Pro Arg Leu Glu Pro Arg Leu Tyr Ser Val Ala Leu
            740                 745                 750

Ala Leu Ala His Ile Ser Ala Arg Gly Pro His Glu Thr Tyr Arg His
        755                 760                 765

Ile Ser Val Ala Leu Pro Arg Ala Leu Ala Ser Glu Arg Ala Ser Asn
    770                 775                 780

Thr His Arg Ala Leu Ala Val Ala Leu Gly Leu Tyr His Ile Ser Leu
785                 790                 795                 800

Glu Ile Leu Glu Leu Glu Gly Leu Ala Ser Asn Gly Leu Asn
                805                 810

<210>  2
<211>  870
<212>  DNA
<213>  Comamonas sp.E6

<400>  2
atggcacgca tcaccgcatc cgttttcacc tcgcacgtgc ctgccatcgg cgccgccatg      60

gacatgggca agacccagga agcctactgg gcgcccctgt tcaagggtta tgacttctcc     120

cgccagtgga tgaaggacaa caagcccgat gtgatcttcc tggtctacaa cgaccacgcc     180

acggccttca gcctggactg cattcccacc ttcgccatcg gcacggctgc ggaattccag 240

cccgccgacg aaggctgggg cccgcgcccc gtgcccaagg tggtcggcca tcccgatctg 300

gccagccaca ttgcccagtc cgtgatccag caggacttcg atctgaccat cgtcaacaag 360

atggacgtgg accacggcct cacggtgcct ctgtcgctga tgtgcggcga gcaggacccc 420

aagaccggct cctggccctg cccggtgatc cccttcgccg tgaacgtggt gcagtatccc 480

gtgcccaccg gccagcgctg cttcaacctg ggccgcgcca tccgcaaggc cgtggagagc 540

tacgaccagg acatcaacgt ccatatctgg ggcacgggtg gcatgagcca ccagctgcag 600

ggtgcgcgcg caggcctgat caacaaggaa tgggacaacc agttcctgga cctgctgatc 660

gagaaccccc acggtctggc gcagatgccg cacatcgact acgtgcgcga agccggctcg 720

gaaggcatcg agctggtgat gtggctgatt gcgcgcggcg ccatgtccga tgtggacggc 780

cccgcaccgc tgcccaaggt ggcgcaccgc ttctaccatg tgcctgcatc gaacaccgcg 840

gtgggccatc tgatcctcga gaatcagtaa 870

<210> 3
<211> 425
<212> PRT
<213> Comamonas sp.E6

<400> 3

Met Glu Thr Ala Leu Ala Leu Glu Gly Leu Leu Tyr Ser Pro Arg Thr
1               5                   10                  15

Tyr Arg Leu Glu Ala Ser Pro Val Ala Leu Pro Arg Gly Leu Tyr Thr
            20                  25                  30

His Arg Ile Leu Glu Ile Leu Glu Pro His Glu Ala Ser Pro Ala Leu
        35                  40                  45

Ala Gly Leu Gly Leu Asn Ser Glu Arg Ala Arg Gly Leu Tyr Ser Gly
    50                  55                  60

Leu Tyr Thr Tyr Arg Thr Arg Pro Leu Glu Ala Ser Asn Gly Leu Asn
65                  70                  75                  80

Pro His Glu Cys Tyr Ser Met Glu Thr Ser Glu Arg Leu Glu Met Glu
                85                  90                  95

Thr Leu Tyr Ser Ala Leu Ala Gly Leu Ala Ser Asn Ala Arg Gly Gly
            100                 105                 110

Leu Ala Arg Gly Pro His Glu Ala Arg Gly Ala Leu Ala Ala Ser Asn
        115                 120                 125

Gly Leu Ala Arg Gly Ala Leu Ala Thr Tyr Arg Leu Glu Ser Glu Arg
    130                 135                 140

Gly Leu Thr Arg Pro Ala Leu Ala Met Glu Thr Ala Arg Gly Gly Leu
145                 150                 155                 160

Gly Leu Gly Leu Asn Leu Tyr Ser Gly Leu Asn Ala Leu Ala Val Ala
                165                 170                 175

Leu Leu Glu Ala Leu Ala Ala Arg Gly Ala Ser Pro Leu Glu Ala Ser
            180                 185                 190

Asn Thr Arg Pro Cys Tyr Ser Met Glu Thr Ala Arg Gly Thr His Arg
        195                 200                 205

Gly Leu Tyr Gly Leu Tyr Ala Ser Asn Ile Leu Glu Thr Tyr Arg Pro
    210                 215                 220

His Glu Leu Glu Ala Leu Ala Leu Tyr Ser Ile Leu Glu Gly Leu Tyr
225                 230                 235                 240

Ala Leu Ala Thr His Arg Ala Ser Pro Gly Leu Tyr Leu Tyr Ser Ser
                245                 250                 255

Glu Arg Pro His Glu Gly Leu Asn Gly Leu Asn Met Glu Thr Ala Leu
            260                 265                 270

Ala Gly Leu Tyr Ser Glu Arg Met Glu Thr Thr His Arg Gly Leu Tyr
        275                 280                 285

Met Glu Thr Thr His Arg Gly Leu Gly Leu Gly Leu Thr Tyr Arg Ala
    290                 295                 300

Arg Gly Ala Leu Ala Met Glu Thr Met Glu Thr Met Glu Thr Gly Leu
305                 310                 315                 320

Tyr Gly Leu Tyr Gly Leu Tyr Ala Arg Gly Ser Glu Arg Ala Leu Ala
                325                 330                 335

Ala Ser Pro Gly Leu Tyr Ala Ser Asn Ala Arg Gly Thr Tyr Arg Val
            340                 345                 350

Ala Leu Gly Leu Tyr Gly Leu Ala Ser Pro Gly Leu Tyr Ala Ser Pro
        355                 360                 365

Ala Leu Ala Gly Leu Asn Ala Leu Ala Ile Leu Glu Ile Leu Glu Ala
    370                 375                 380

Arg Gly Gly Leu Asn Pro Arg Gly Leu Asn Gly Leu Tyr Ser Glu Arg
385                 390                 395                 400

Ala Leu Ala Gly Leu Tyr Ala Ser Asn Gly Leu Asn Ala Ser Asn Gly
                405                 410                 415

Leu Gly Leu Gly Leu Tyr Ala Ser Asn
            420                 425

<210>  4
<211>  450
<212>  DNA
<213>  Comamonas sp.E6

<400>  4
atggctttgg aaaaaccgta tctggacgtg cccggcacca tcattttcga tgccgagcag      60

tcccgcaagg gctactggct caaccagttc tgcatgagcc tgatgaaggc cgagaaccgc     120

gagcgctttc gcgccaacga gcgtgcctat ctggacgagt gggcgatgac cgaggagcag     180

aagcaggccg tgctggcgcg tgacctgaac tggtgcatgc gcaccggcgg caatatctac     240

ttcctggcca agattggcgc caccgacggc aagagcttcc agcagatggc gggctccatg     300

accggcatga ccgaagaaga gtaccgcgcc atgatgatgg gcggcggccg ctctgccgat     360

ggcaatcgct atgtgggcga ggacggtgat gcgcaggcgc atcgccagcc ccagggcagc     420

gcaggcaacc agaacaagga aggcaactaa                                      450

<210>  5
<211>  26
<212>  DNA
<213>  Artificial Sequence

<220>
<223>  aF Primer

<400>  5
atgwssctga tgaarscsga raaccg                                          26

<210>  6
<211>  27
<212>  DNA
<213>  Artificial Sequence

<220>
<223>  bF Primer

<400>  6
gtswtcctgg tstayaacga ycaygcy                                         27

<210>  7
<211>  25
<212>  DNA
<213>  Artificial Sequence

<220>
<223>  bR Primer

<400>  7
ccctgmarct grtggctcat gccgc                                           25

<210> 8
<211> 5386
<212> DNA
<213> Comamonas sp. E6

<400> 8
atgatcattg acgtacacgg tcactacacc acggcgcctg cggctctggg cgcatggcgc 60

gatctgcaga tcgccggcct caaggacccg agcaagaccc cgtcggtggc cgatctgaag 120

atcagcgacg atgaaatccg cgagaccatc gaaaccaatc agctgcgcct gatgaaggag 180

cgtggttccg atctgaccat cttcagcccc cgtgcctcgt tcatggcgca ccacatcggt 240

gacttccaga cctccagcac ctgggccgcc atctgcaacg agctgtgctt ccgcgtcagc 300

gagctgttcc ccgaccactt cattcccgcc gccatgctgc cccagtcgcc cggcgtggac 360

cctgcaacct gcattcccga gctggtcaag tgcgttgagc aatatggcaa cgtgggcctg 420

aacctgaacc ccgatccctc gggcggtcac tggacttcgc ctccgctgtc cgacaagagc 480

tggtacccca tctacgaaaa gatggtggag tacgacatcc ccgcgatgat ccacgtctcc 540

accagctgca atagctgctt ccacaccacg ggcagccact atctgaatgc cgacaccacg 600

gccttcatgc agtgcctgac ttcggatctg ttcaaggact tcccgaccct gaagttcctg 660

attccccatg gcggcggcgc cgtgccttac cactggggcc gtttccgcgg tctggcgcag 720

gagatgaaga agccgctgct ggaagagcat ttgctcaaca acatctactt cgacacctgc 780

gtctaccacc agccgggcat caacctgctg acggaagtga ttccgaccaa gaacattctg 840

ttcgccagcg aaatgatcgg cgccgtgcgc ggcatcgatc cgcagaccgg tcactactac 900

gacgacacca agcgctacat cgaagccacg cagaacctga cggccgatga aaagcacgcc 960

gtctacgaag gcaatgcccg ccgcgtgttc acgcgcctgg acaaggcgct caaggccaag 1020

ggtctgtaac ttctaaaaaa gagagctgct ggcgcacggt atgaaaggtt ttcaataaga 1080

aaactattca gatcttatga atagcaagcg ctaacagctc actttttaga ttcaataacg 1140

tcaaagcaaa gaggtatttc catgtacgaa ctgggagttg tctaccgcaa tatccagcgc 1200

gccgaccgcg ctgctgctga cggcctggcc gccctgggct ctgccaccgt gcacgaggcc 1260

atgggccgcg ttggtctgct caagccctat atgcgcccca tctatgccgg caagcaggtc 1320

tcgggtacgg ccgtcacggt gctgctgcag cccggcgaca actggatgat gcatgtggct 1380

gccgagcaga ttcagcccgg cgatatcgtg gtcgctgccg tcaccgccga gtgctccgac 1440

ggctacttcg gcgacctgct ggccaccagc ttccaggcgc gcggcgcacg cgcgctgatc 1500

atcgatgccg gcgtgcgcga cgtgaagacg ctgcaggaga tggacttccc ggtctggagc 1560

aaggccatct cttccaaggg cacgatcaag gccaccctgg gctcggtcaa catccccatc 1620

gtctgcgccg gcatgctggt cacgcccggt gacgtgatcg tggccgacga cgacggcgtg 1680

gtctgcgtgc ccgccgcgcg tgccgtggaa gtgctggccg ctgcccagaa gcgcgaaagc 1740

ttcgaaggcg aaaagcgcgc caagctggct tcgggcgtcc tgggcctgga tatgtacaag 1800

atgcgcgagc ccctggaaaa ggccggcttg cgctatgtgg attaactccc cctgagcggc 1860

tgcgccgctt ccccctctct cgctgcgcgg gaaggggacg acaccctcgg tgcggggcgg 1920

cccttcctcg gtgtctctga gatgtggcag cgccagtttc atggatggtg ggaagtgcgc 1980

agcgcacggt gcaatcaaca attgaggagc aaacagatga gcgcctttga aaaaaccccc 2040

ggctggctgg actggtatgc caaccccagc aagccccagt tcaagctgcc tgccggcgct 2100

gtggatgcgc actgccatgt gttcggtccc ggtaacgagt tccccttcgc ccccgagcgc 2160

aagtacaccc cctgcgacgc cagcaaggcc cagctgtatg cgctgcgcga ccatctgggt 2220

tttgcgcgca atgtggtggt gcaggccacc tgccacggtg cggacaaccg cgccatggtc 2280

gatgcctgca agtcctcggg cggcaaggcc cgcggcgtgg ccacggtcaa gcgctccatc 2340

agcgatgccg aactgcagga gctgcatgac gccggcgtgc gcggcgtgcg tttcaacttc 2400

gtcaagcgtc tggtggactt tacgcccaag gacgagctga tggagatcgc cggtcgcatc 2460

gccaagctgg gctggcatgt ggtgatctat ttcgaagccg tggatctgcc cgagctgtgg 2520

gacttcttca ccgcgctgcc caccaccgtg gtggtcgacc acatgggccg ccccgacgtg 2580

accaagggcg tggacagcga ggagttcgcc ctgttcttga agttcatgcg cgagcacaag 2640

aatgtctgga gcaaggtttc ctgccccgag cgcctgtccg tctccggccc caaggcgctt 2700

catggtgagc agaacgccta ccaggacgtg gtgcctttcg cgcgccgcgt ggtcgaggag 2760

ttccccgagc gcgtgctctg gggcacggac tggccgcacc ccaacctgaa ggaccacatg 2820

cccgacgacg gcctgctggt ggacttcatt cctcatatcg ctcctaccgc gcagctgcag 2880

caaaagctgc tggtggacaa ccccatgcgt ctgtactggc ccgaagaggt ctgacggagt 2940

tccggcgaca catacacccg gaaacaggcc gcagccttgt cgcatgggct gcgccatccg 3000

taacgaggag aatttatggc tttggaaaaa ccgtatctgg acgtgcccgg caccatcatt 3060

ttcgatgccg agcagtcccg caagggctac tggctcaacc agttctgcat gagcctgatg 3120

aaggccgaga accgcgagcg ctttcgcgcc aacgagcgtg cctatctgga cgagtgggcg 3180

atgaccgagg agcagaagca ggccgtgctg gcgcgtgacc tgaactggtg catgcgcacc 3240

ggcggcaata tctacttcct ggccaagatt ggcgccaccg acggcaagag cttccagcag 3300

atggcgggct ccatgaccgg catgaccgaa gaagagtacc gcgccatgat gatgggcggc 3360

ggccgctctg ccgatggcaa tcgctatgtg ggcgaggacg gtgatgcgca ggcgcatcgc 3420

cagccccagg gcagcgcagg caaccagaac aaggaaggca actaagacat ggcacgcatc 3480

accgcatccg ttttcacctc gcacgtgcct gccatcggcg ccgccatgga catgggcaag 3540

acccaggaag cctactgggc gcccctgttc aagggttatg acttctcccg ccagtggatg 3600

aaggacaaca agcccgatgt gatcttcctg gtctacaacg accacgccac ggccttcagc 3660

ctggactgca ttcccacctt cgccatcggc acggctgcgg aattccagcc cgccgacgaa 3720

ggctggggcc cgcgccccgt gcccaaggtg gtcggccatc ccgatctggc cagccacatt 3780

gcccagtccg tgatccagca ggacttcgat ctgaccatcg tcaacaagat ggacgtggac 3840

cacggcctca cggtgcctct gtcgctgatg tgcggcgagc aggaccccaa gaccggctcc 3900

tggccctgcc cggtgatccc cttcgccgtg aacgtggtgc agtatcccgt gcccaccggc 3960

cagcgctgct tcaacctggg ccgcgccatc cgcaaggccg tggagagcta cgaccaggac 4020

atcaacgtcc atatctgggg cacgggtggc atgagccacc agctgcaggg tgcgcgcgca 4080

ggcctgatca acaaggaatg ggacaaccag ttcctggacc tgctgatcga gaacccccac 4140

ggtctggcgc agatgccgca catcgactac gtgcgcgaag ccggctcgga aggcatcgag 4200

ctggtgatgt ggctgattgc gcgcggcgcc atgtccgatg tggacggccc cgcaccgctg 4260

cccaaggtgg cgcaccgctt ctaccatgtg cctgcatcga acaccgcggt gggccatctg 4320

atcctcgaga atcagtaaac gtttcaccac gttcgctgct tcgcgtattc actgccccct 4380

gtggggggct tcgcctcctt gaggcggctc tacggagatc ccaatcatga gcaagaccat 4440

caaagtagcg ctggctggcg caggtgcctt cggcatcaag cacctggacg gcatcaagaa 4500

catcgacggc gtggaagtcg tctccctggt cggtcgccgc tttgaccaga ccaaggaagt 4560

ggccgacaaa tacggcatca agcatgtggc aaccgatctg gccgaaagcc tggcgctgcc 4620

cgaggtcgat gccgtgatcc tttgcacgcc cacgcagatg cacgccgagc aggccattgc 4680

ctgcatgaag gccggcaagc atgtgcaggt cgagattcct ctggccgatg ccctgaagga 4740

cgcccaggaa gtggccgagc tgcaaaagca gaccggtctg gtggccatgg tgggtcacac 4800

ccgccgcttc aaccccagcc accagtgggt gcacaagaag atagcagccg gcgagttcaa 4860

catccagcag atggatgtgc aaacctactt cttccgccgc accaatatga acgcgctggg 4920

ccaggcccgc agctggaccg accacctgct gtggcaccat gccgcccaca ccgtggacct 4980

gttcgcctac caggccggca gccccatcgt caaggccaac gccgtgcaag gcccgattca 5040

caaggatctg ggcatcgcca tggacatgag catccagctc aaggccgcca acggcgcgat 5100

ctgcacgctg agcctgtcgt tcaacaatga cggccctctg ggtaccttct tccgctacat 5160

cggcgacacc ggcacctatc tggcccgcta cgacgatctg tacaccggca aggacgagaa 5220

gatcgacgtg tcccaggtcg atgtgtccat gaacggcatc gagctgcagg accgcgaatt 5280

cttcgccgcc atccgtgaag gccgcgagcc caactccagc gtgcagcagg tgttcaactg 5340

ctacaaggtc ttgcacgacc tggagcagca actcaacgcc gaataa 5386

<210> 9
<211> 768
<212> DNA
<213> Comamonas sp.

<400> 9
atgcaggaca agaactttgt ggaatcgctg cgcaagggat tgggggtact gacttgcttt 60

gaccgtcggc atacccggct gacgctgtca gaggtagcca ggctcacgca gtccacgcca 120

gcatccgcca gacgttcgct cagcacactg gtacagcttg gctatctaga gagcgacggc 180

aaactgttct ggatgcagcc caaatcgctg ctgatcgcct attcatttct gtcatcgcgc 240

cccatgcctg cattggccca gccactactg gatgcactgt cggagcgcac cagagaatcc 300

gcttcgcttg gtactttgtt ggaggacgat gccatcatca ttggtcgttc gaccgcacgg 360

cgcagcttga gcacgggcct aggaatagga tctaggttgc cggtgtactg ctctgcgatt 420

ggtcggatgc tgttgtcagg actcccccaa caggaggcgc gtgcaaggct agagatgatc 480

gagcgggtgg cactgacccc tcatacggtg actgacttgg aggagctgct aggtctgctt 540

gaaacttgcc ggcaatcagg gtggtcatgc agcgacggag agctggagct gggggtgcgc 600

tctatggcag cgccagtgcg cgaccctcaa ggcaacacaa ttgctgccat gagcattgct 660

gttagggcag agagactcag catgagtgag ttcaaggaga cttttttgat accgctgaag 720

cgcgctcgca atgagttgga aaaaaagcta tatccgcagg ggttgtag 768

<210> 10
<211> 969
<212> DNA
<213> Comamonas sp.

<400> 10
atgcgcaacg aatctattcg cagacgcgaa gcattgatcg gcattgccgc tgcggtcgct 60

gcaacaggta gcctggctca gtcaaatcaa cctctcaaga tcgtcgtgcc tttctcagcg 120

ggaggtaccg ccgatgtact gccgcgccta gtagccgaaa aaatccgcgc ggactacgca 180

ggtggcgtga tcattgaaaa caagcccggg gctggtggaa acatcggtgc cgatctagtg 240

ttccgggcac cgccagacgg aatgactgtg ctagcctccc ctccggggcc catcgccatc 300

aaccacaacc tctatcaaaa gctcagcttc gacccgactc gctgggtacc ggtgacgatt 360

ttggccacag tgcccaacgt cctggtcatc aacccgaagc tgcctgttaa gtcacttggc 420

gagttcatcg catacgccaa agccaatccc aaaaaagtga ctgtggcgac gcaaggcgac 480

ggctccacat cccacctgac tgctgccatg tttatgcagc tgactggcac tgaactgact 540

gtcatccctt acaagggaac agccccggca ctgattgact tgatcggcgg caatgtggat 600

gtgtttttcg acaacatcag ctcgtcggcc acctaccacc aggccggcaa ggtgcgcatt 660

ctggccgttg ccgacgagca gcgctctcaa atattgcccc aggtccccac cttcgccgag 720

cagcaatggc ccgccatgca agccgtgact ttcttttctg ttgtggctcc cccaggcacg 780

agtgcagaaa tcgctcagaa gcttcagaag cagatggccc tggctctgtc atcgaacgac 840

atccgcaagc actttcagga acaaggtgcc gtgccttgtg gttgggaccc gtccaagact 900

gctcagttca tccgccagga aaccgagaag tggaagaagg tgctgaaggc cgccaacgtc 960

aagctctaa 969

<210> 11
<211> 1242
<212> DNA
<213> Comamonas sp.

<400> 11
atgcaagaat ccatcatcca gtggcatggg gccactaata cgcgcgtgcc ttttggtatc 60

tataccgaca cagccaatgc tgatcaggaa cagcagcgca tctatcgcgg cgaggtctgg 120

aactacttgt gcctggaatc tgaaattccc ggggccggtg atttccgcac tacctttgcc 180

ggtgaaacac cgatagttgt cgtacgggat gccgaccagg aaatctacgc cttcgagaac 240

cgctgcgcgc atcgcggcgc tctcatcgct ctggagaaat cgggccgtac ggatagtttc 300

cagtgcgtct atcacgcctg gagctacaac cgacagggag atctgaccgg cgttgccttc 360

gagaaaggtg tcaagggcca gggtggcatg ccggcctcat tctgcaaaga agagcatggc 420

ccgcgcaagc tccgcgtggc tgtcttttgc ggtttggtct ttggcagttt ttccgaggac 480

gtgcccagca ttgaggatta ccttggccct gagatttgcg agcgcataga gcgcgtgctg 540

cacaagcccg tagaagtcat cggtcgcttc acgcaaaagc tgcctaacaa ctggaagctc 600

tacttcgaga acgtgaagga cagctatcac gccagcctcc tgcatatgtt cttcaccacc 660

ttcgagctga atcgcctctc acaaaaaggc ggtgtcatcg tcgacgagtc gggtggccac 720

catgtgagct attccatgat cgatcgcggc gccaaagacg actcgtacaa ggaccaggcc 780

atccgctccg acaacgagcg ttaccggctc aaagatccta gccttctaga gggctttgag 840

gagttcgagg acggcgtgac cctgcagatc ctttctgtgt tccctggctt tgtgctgcag 900

cagattcaga acagcatcgc cgtgcgtcag ttgctgccca agagcatctc cagctcggaa 960

ctcaactgga cctatcttgg ctatgcagat gacagtgccg agcaacgcaa ggtcagactc 1020

aaacaggcca accttatcgg cccggccgga ttcatttcca tggaagacgg agctgtcggt 1080

ggattcgtgc agcgtggcat cgcaggcgct gccaaccttg atgcagtcat cgagatgggc 1140

ggagaccacg aaggctctag cgagggccgc gccacggaaa cctcggtacg cggcttttgg 1200

aaggcctacc gcaagcatat gggacaggag atgcaagcat ga 1242

<210> 12
<211> 465
<212> DNA
<213> Comamonas sp.

<400> 12
atgatcaatg aaattcaaat cgcggccttc aatgccgcct acgcgaagac catagacagt 60

gatgcaatgg agcaatggcc aacctttttc accaaggatt gccactattg cgtcaccaat 120

gtcgacaacc atgatgaggg acttgctgcc ggcattgtct gggcggattc gcaggacatg 180

ctcaccgacc gaatttctgc gctgcgcgaa gccaatatct acgagcgcca ccgctatcgc 240

catatcctgg gtctgccttc gatccagtca ggcgatgcaa cacaggccag cgcttccact 300

ccgttcatgg tgctgcgcat catgcataca ggggaaacag aggtctttgc cagcggtgag 360

tacctcgaca aattcaccac gatcgatggc aagttacgtc tgcaagaacg catcgcggtt 420

tgcgacagca cggtgacgga cacgctgatg gcattgccgc tatga 465

<210> 13
<211> 948
<212> DNA
<213> Comamonas sp.

<400> 13
atgacaatag tgcaccgtag attggctttg gccatcggcg atccccacgg tattggccca 60

gaaatcgcac tgaaagctct ccagcagctg tctgtcaccg aaaggtctct tatcaaggtc 120

tatggacctt ggagcgctct cgagcaagcc gcacgggttt gcgaaatgga gccgcttctt 180

caagacatcg ttcacgagga agccggcaca cttacacaac cagttcaatg gggagaaatc 240

accccgcagg ctggtctatc tacggtgcaa tccgcaacag cggctatccg agcgtgcgaa 300

aacggcgaag tcgatgccgt cattgcctgc cctcaccatg aaacggccat tcaccgcgca 360

ggcatagcgt tcagcggcta cccatctttg ctcgccaatg ttcttggcat gaacgaagac 420

caggtattcc tgatgctggt gggggctggc ctgcgcatag tgcatgtcac tttgcatgaa 480

agcgtgcgca gcgcattgga gcggctctct cctcagttgg tggtcaacgc ggcgcaggct 540

gccgtgcaga catgcacctt actcggagtg cctaaaccaa aagtcgctgt attcgggatc 600

aaccctcatg catctgaagg acagttgttc ggcctggagg actcccagat caccgttccc 660

gctgtcgaga cactgcgcaa gcgcggccta gcagtagacg gccccatggg agctgacatg 720

gttctggcac agcgcaagca cgacctgtat gtagccatgc tgcacgatca gggccatatc 780

cccatcaagc tgctggcacc taacggagcc agcgcactat ctatcggtgg cagggtggtg 840

ctttccagcg tgggccatgg cagcgccatg gacattgccg gccgtggcgt ggctgacgcc 900

acggccctcc tacgcacaat agccctactc ggagcccaac cggtctga 948

<210> 14
<211> 1011
<212> DNA
<213> Comamonas sp.

<400> 14
atgaaccacc agatccatat ccacgactcc gatatcgcgt tcccctgcgc gcccgggcaa 60

tccgtactgg atgcagctct gcaggccggc atcgagctgc cctattcctg ccgcaaaggt 120

agctgtggca actgtgcgag tacgctgctc gacggaaata ttgcctcctt caatggcatg 180

gccgtgcgaa acgaactctg cgcctcggaa caagtgctgc tgtgcggctg cactgcagcc 240

agcgatatac gtatccaccc gagctccttt cgccgtctcg acccggaagc ccgaaaacgt 300

tttacggcca aggtgtacag caatacactg gcggcacccg atgtctcgct gctgcgcctg 360

cgcctgcctg tgggcaagcg cgccaaattt gaagccggcc aatacctgct gattcacctc 420

gacgacgggg aaagccgcag ctactctatg gccaatccac cccatgagag cgatggcatc 480

acattgcatg tcaggcatgt acctggtggt cgcttcagca ctatcgttca gcagttgaag 540

tctggtgaca cattggatat cgaactgcca ttcggcagca tcgcactgaa gcctgatgac 600

gcaaggcccc tgatttgcgt tgcgggtggc acgggatttg cgcccattaa atccgttctt 660

gatgacttag ccaaacgcaa ggttcagcgc gacatcacgc tgatctgggg ggctcgcaac 720

ccctcgggcc tgtatcttcc tagcgccatc gacaagtggc gcaaagtctg gccacagttt 780

cgctacattg cagccatcac cgacctaggc gatatgcctg cggatgctca cgcaggtcgg 840

gtggatgacg cgctacgcac tcactttggc aacctgcacg atcatgtggt gcactgctgt 900

ggctcaccag ctctggttca atcagtgcgc acagccgctt ccgatatggg cctgcttgca 960

caggacttcc acgcggatgt ttttgcgaca ggcccgactg gtcaccacta g 1011

<210> 15
<211> 720
<212> DNA
<213> Pseudomonas putida KT2440

<400> 15
atgcccgccc aggacaacag ccgcttcgtg atccgtgatc gcaactggca ccctaaagcc 60

cttacgcctg actacaagac ctccgttgcc cgctcgccgc gccaggcact ggtcagcatt 120

ccgcagtcga tcagcgaaac cactggtccg gacttttccc atctgggctt cggcgcccac 180

gaccatgacc tgctgctgaa cttcaataac ggtggcctgc ccattggcga gcgcatcatc 240

gtcgccggcc gtgtcgtcga ccagtacggc aagcctgtgc cgaacacttt ggtggagatg 300

tggcaagcca acgccggcgg ccgctatcgc cacaagaacg atcgctacct ggcgcccctg 360

gacccgaact tcggtggtgt tgggcggtgt ctgaccgacc gtgacggcta ttacagcttc 420

cgcaccatca agccgggccc gtacccatgg cgcaacggcc cgaacgactg gcgcccggcg 480

catatccact tcgccatcag cggcccatcg atcgccacca agctgatcac ccagttgtac 540

ttcgaaggtg acccgctgat cccgatgtgc ccgatcgtca agtcgatcgc caacccgcaa 600

gccgtgcagc agttgatcgc caagctcgac atgagcaacg ccaacccgat ggactgcctg 660

gcctaccgct ttgacatcgt gctgcgcggc cagcgcaaga cccacttcga aaactgctga 720

<210> 16
<211> 606
<212> DNA
<213> Pseudomonas putida KT2440

<400> 16

atgccaatcg aactgctgcc ggaaacccct tcgcagactg ccggccccta cgtgcacatc 60

ggcctggccc tggaagccgc cggcaacccg acccgcgacc aggaaatctg gaactgcctg 120

gccaagccag acgccccggg cgagcacatt ctgctgatcg gccacgtata tgacggaaac 180

ggccacctgg tgcgcgactc gttcctggaa gtgtggcagg ccgacgccaa cggtgagtac 240

caggatgcct acaacctgga aaacgccttc aacagctttg gccgcacggc taccaccttc 300

gatgccggtg agtggacgct gcaaacggtc aagccgggtg tggtgaacaa cgctgctggc 360

gtgccgatgg cgccgcacat caacatcagc ctgtttgccc gtggcatcaa catccacctg 420

cacacgcgcc tgtatttcga tgatgaggcc caggccaatg ccaagtgccc ggtgctcaac 480

ctgatcgagc agccgcagcg gcgtgaaacc ttgattgcca agcgttgcga agtggatggg 540

aagacggcgt accgctttga tatccgcatt cagggggaag gggagaccgt cttcttcgac 600

ttctga 606

SEQUENCE LISTING

<110> TOYOTA INDUSTRIES CORPORATION/ Tokyo University of Agriculture and
      Technology/ Nagaoka University of Technology/ Forestry and Forest
      Products Research Institute

<120> Mutant, Recombinat Plasmid, Transformant and Method for
      Producing 3-Carboxymuconolactone

<130> V576-PCT

<160> 16

<170> PatentIn version 3.4

<210> 1
<211> 814
<212> PRT
<213> Comamonas sp. E6

<400> 1

Met Glu Thr Ala Leu Ala Ala Arg Gly Ile Leu Glu Thr His Arg Ala
1               5                   10                  15

Leu Ala Ser Glu Arg Val Ala Leu Pro His Glu Thr His Arg Ser Glu
            20                  25                  30

Arg His Ile Ser Val Ala Leu Pro Arg Ala Leu Ala Ile Leu Glu Gly
        35                  40                  45

Leu Tyr Ala Leu Ala Ala Leu Ala Met Glu Thr Ala Ser Pro Met Glu
    50                  55                  60

Thr Gly Leu Tyr Leu Tyr Ser Thr His Arg Gly Leu Asn Gly Leu Ala
65                  70                  75                  80

Leu Ala Thr Tyr Arg Thr Arg Pro Ala Leu Ala Pro Arg Leu Glu Pro
                85                  90                  95

His Glu Leu Tyr Ser Gly Leu Tyr Thr Tyr Arg Ala Ser Pro Pro His
            100                 105                 110

Glu Ser Glu Arg Ala Arg Gly Gly Leu Asn Thr Arg Pro Met Glu Thr
        115                 120                 125

Leu Tyr Ser Ala Ser Pro Ala Ser Asn Leu Tyr Ser Pro Arg Ala Ser
    130                 135                 140

Pro Val Ala Leu Ile Leu Glu Pro His Glu Leu Glu Val Ala Leu Thr
145                 150                 155                 160

Tyr Arg Ala Ser Asn Ala Ser Pro His Ile Ser Ala Leu Ala Thr His
                165                 170                 175

Arg Ala Leu Ala Pro His Glu Ser Glu Arg Leu Glu Ala Ser Pro Cys
            180                 185                 190

Tyr Ser Ile Leu Glu Pro Arg Thr His Arg Pro His Glu Ala Leu Ala
        195                 200                 205

Ile Leu Glu Gly Leu Tyr Thr His Arg Ala Leu Ala Ala Leu Ala Gly
    210                 215                 220

Leu Pro His Glu Gly Leu Asn Pro Arg Ala Leu Ala Ala Ser Pro Gly
225                 230                 235                 240

Leu Gly Leu Tyr Thr Arg Pro Gly Leu Tyr Pro Arg Ala Arg Gly Pro
                245                 250                 255

Arg Val Ala Leu Pro Arg Leu Tyr Ser Val Ala Leu Val Ala Leu Gly
            260                 265                 270

Leu Tyr His Ile Ser Pro Arg Ala Ser Pro Leu Glu Ala Leu Ala Ser
        275                 280                 285

Glu Arg His Ile Ser Ile Leu Glu Ala Leu Ala Gly Leu Asn Ser Glu
    290                 295                 300

Arg Val Ala Leu Ile Leu Glu Gly Leu Asn Gly Leu Asn Ala Ser Pro
305                 310                 315                 320

Pro His Glu Ala Ser Pro Leu Glu Thr His Arg Ile Leu Glu Val Ala
                325                 330                 335

Leu Ala Ser Asn Leu Tyr Ser Met Glu Thr Ala Ser Pro Val Ala Leu
            340                 345                 350

Ala Ser Pro His Ile Ser Gly Leu Tyr Leu Glu Thr His Arg Val Ala
        355                 360                 365

Leu Pro Arg Leu Glu Ser Glu Arg Leu Glu Met Glu Thr Cys Tyr Ser
    370                 375                 380

Gly Leu Tyr Gly Leu Gly Leu Asn Ala Ser Pro Pro Arg Leu Tyr Ser
385                 390                 395                 400

Thr His Arg Gly Leu Tyr Ser Glu Arg Thr Arg Pro Pro Arg Cys Tyr
                405                 410                 415

Ser Pro Arg Val Ala Leu Ile Leu Glu Pro Arg Pro His Glu Ala Leu
            420                 425                 430

Ala Val Ala Leu Ala Ser Asn Val Ala Leu Val Ala Leu Gly Leu Asn
        435                 440                 445

Thr Tyr Arg Pro Arg Val Ala Leu Pro Arg Thr His Arg Gly Leu Tyr
    450                 455                 460

Gly Leu Asn Ala Arg Gly Cys Tyr Ser Pro His Glu Ala Ser Asn Leu
465                 470                 475                 480

Glu Gly Leu Tyr Ala Arg Gly Ala Leu Ala Ile Leu Glu Ala Arg Gly
                485                 490                 495

Leu Tyr Ser Ala Leu Ala Val Ala Leu Gly Leu Ser Glu Arg Thr Tyr
            500                 505                 510

Arg Ala Ser Pro Gly Leu Asn Ala Ser Pro Ile Leu Glu Ala Ser Asn
        515                 520                 525

Val Ala Leu His Ile Ser Ile Leu Glu Thr Arg Pro Gly Leu Tyr Thr
    530                 535                 540

His Arg Gly Leu Tyr Gly Leu Tyr Met Glu Thr Ser Glu Arg His Ile
545                 550                 555                 560

Ser Gly Leu Asn Leu Glu Gly Leu Asn Gly Leu Tyr Ala Leu Ala Ala
                565                 570                 575

Arg Gly Ala Leu Ala Gly Leu Tyr Leu Glu Ile Leu Glu Ala Ser Asn
            580                 585                 590

Leu Tyr Ser Gly Leu Thr Arg Pro Ala Ser Pro Ala Ser Asn Gly Leu
        595                 600                 605

Asn Pro His Glu Leu Glu Ala Ser Pro Leu Glu Leu Glu Ile Leu Glu
    610                 615                 620

Gly Leu Ala Ser Asn Pro Arg His Ile Ser Gly Leu Tyr Leu Glu Ala
625                 630                 635                 640

Leu Ala Gly Leu Asn Met Glu Thr Pro Arg His Ile Ser Ile Leu Glu
                645                 650                 655

Ala Ser Pro Thr Tyr Arg Val Ala Leu Ala Arg Gly Gly Leu Ala Leu
            660                 665                 670

Ala Gly Leu Tyr Ser Glu Arg Gly Leu Gly Leu Tyr Ile Leu Glu Gly
        675                 680                 685

Leu Leu Glu Val Ala Leu Met Glu Thr Thr Arg Pro Leu Glu Ile Leu
    690                 695                 700

Glu Ala Leu Ala Ala Arg Gly Gly Leu Tyr Ala Leu Ala Met Glu Thr
705                 710                 715                 720

Ser Glu Arg Ala Ser Pro Val Ala Leu Ala Ser Pro Gly Leu Tyr Pro
                725                 730                 735

Arg Ala Leu Ala Pro Arg Leu Glu Pro Arg Leu Tyr Ser Val Ala Leu
            740                 745                 750

Ala Leu Ala His Ile Ser Ala Arg Gly Pro His Glu Thr Tyr Arg His
        755                 760                 765

Ile Ser Val Ala Leu Pro Arg Ala Leu Ala Ser Glu Arg Ala Ser Asn
    770                 775                 780

Thr His Arg Ala Leu Ala Val Ala Leu Gly Leu Tyr His Ile Ser Leu
785                 790                 795                 800

Glu Ile Leu Glu Leu Glu Gly Leu Ala Ser Asn Gly Leu Asn
                805                 810

<210> 2
<211> 870
<212> DNA
<213> Comamonas sp. E6

<400> 2
atggcacgca tcaccgcatc cgttttcacc tcgcacgtgc ctgccatcgg cgccgccatg      60

gacatgggca agacccagga agcctactgg gcgcccctgt tcaagggtta tgacttctcc     120

cgccagtgga tgaaggacaa caagcccgat gtgatcttcc tggtctacaa cgaccacgcc     180

acggccttca gcctggactg cattcccacc ttcgccatcg gcacggctgc ggaattccag     240

cccgccgacg aaggctgggg cccgcgcccc gtgcccaagg tggtcggcca tcccgatctg     300

gccagccaca ttgcccagtc cgtgatccag caggacttcg atctgaccat cgtcaacaag     360

atggacgtgg accacggcct cacggtgcct ctgtcgctga tgtgcggcga gcaggacccc     420

aagaccggct cctggccctg cccggtgatc cccttcgccg tgaacgtggt gcagtatccc     480

gtgcccaccg gccagcgctg cttcaacctg ggccgcgcca tccgcaaggc cgtggagagc     540

tacgaccagg acatcaacgt ccatatctgg ggcacgggtg gcatgagcca ccagctgcag     600

ggtgcgcgcg caggcctgat caacaaggaa tgggacaacc agttcctgga cctgctgatc 660

gagaaccccc acggtctggc gcagatgccg cacatcgact acgtgcgcga agccggctcg 720

gaaggcatcg agctggtgat gtggctgatt gcgcgcggcg ccatgtccga tgtggacggc 780

cccgcaccgc tgcccaaggt ggcgcaccgc ttctaccatg tgcctgcatc gaacaccgcg 840

gtgggccatc tgatcctcga gaatcagtaa 870

<210> 3
<211> 425
<212> PRT
<213> Comamonas sp. E6

<400> 3

Met Glu Thr Ala Leu Ala Leu Glu Gly Leu Leu Tyr Ser Pro Arg Thr
1               5                   10                  15

Tyr Arg Leu Glu Ala Ser Pro Val Ala Leu Pro Arg Gly Leu Tyr Thr
            20                  25                  30

His Arg Ile Leu Glu Ile Leu Glu Pro His Glu Ala Ser Pro Ala Leu
        35                  40                  45

Ala Gly Leu Gly Leu Asn Ser Glu Arg Ala Arg Gly Leu Tyr Ser Gly
    50                  55                  60

Leu Tyr Thr Tyr Arg Thr Arg Pro Leu Glu Ala Ser Asn Gly Leu Asn
65                  70                  75                  80

Pro His Glu Cys Tyr Ser Met Glu Thr Ser Glu Arg Leu Glu Met Glu
                85                  90                  95
Thr Leu Tyr Ser Ala Leu Ala Gly Leu Ala Ser Asn Ala Arg Gly Gly
            100                 105                 110
Leu Ala Arg Gly Pro His Glu Ala Arg Gly Ala Leu Ala Ala Ser Asn
        115                 120                 125
Gly Leu Ala Arg Gly Ala Leu Ala Thr Tyr Arg Leu Glu Ser Glu Arg
    130                 135                 140
Gly Leu Thr Arg Pro Ala Leu Ala Met Glu Thr Ala Arg Gly Gly Leu
145                 150                 155                 160
Gly Leu Gly Leu Asn Leu Tyr Ser Gly Leu Asn Ala Leu Ala Val Ala
                165                 170                 175
Leu Leu Glu Ala Leu Ala Ala Arg Gly Ala Ser Pro Leu Glu Ala Ser
            180                 185                 190
Asn Thr Arg Pro Cys Tyr Ser Met Glu Thr Ala Arg Gly Thr His Arg
        195                 200                 205
Gly Leu Tyr Gly Leu Tyr Ala Ser Asn Ile Leu Glu Thr Tyr Arg Pro
    210                 215                 220

His Glu Leu Glu Ala Leu Ala Leu Tyr Ser Ile Leu Glu Gly Leu Tyr
225                 230                 235                 240

Ala Leu Ala Thr His Arg Ala Ser Pro Gly Leu Tyr Leu Tyr Ser Ser
                245                 250                 255

Glu Arg Pro His Glu Gly Leu Asn Gly Leu Asn Met Glu Thr Ala Leu
            260                 265                 270

Ala Gly Leu Tyr Ser Glu Arg Met Glu Thr Thr His Arg Gly Leu Tyr
        275                 280                 285

Met Glu Thr Thr His Arg Gly Leu Gly Leu Gly Leu Thr Tyr Arg Ala
    290                 295                 300

Arg Gly Ala Leu Ala Met Glu Thr Met Glu Thr Met Glu Thr Gly Leu
305                 310                 315                 320

Tyr Gly Leu Tyr Gly Leu Tyr Ala Arg Gly Ser Glu Arg Ala Leu Ala
                325                 330                 335

Ala Ser Pro Gly Leu Tyr Ala Ser Asn Ala Arg Gly Thr Tyr Arg Val
            340                 345                 350

Ala Leu Gly Leu Tyr Gly Leu Ala Ser Pro Gly Leu Tyr Ala Ser Pro
        355                 360                 365

Ala Leu Ala Gly Leu Asn Ala Leu Ala Ile Leu Glu Ile Leu Glu Ala
    370                 375                 380

Arg Gly Gly Leu Asn Pro Arg Gly Leu Asn Gly Leu Tyr Ser Glu Arg
385                 390                 395                 400

Ala Leu Ala Gly Leu Tyr Ala Ser Asn Gly Leu Asn Ala Ser Asn Gly
                405                 410                 415

Leu Gly Leu Gly Leu Tyr Ala Ser Asn
            420                 425

<210>  4
<211>  450
<212>  DNA
<213>  Comamonas sp. E6

<400>  4
atggctttgg aaaaaccgta tctggacgtg cccggcacca tcattttcga tgccgagcag      60

tcccgcaagg gctactggct caaccagttc tgcatgagcc tgatgaaggc cgagaaccgc     120

gagcgctttc gcgccaacga gcgtgcctat ctggacgagt gggcgatgac cgaggagcag     180

aagcaggccg tgctggcgcg tgacctgaac tggtgcatgc gcaccggcgg caatatctac     240

ttcctggcca agattggcgc caccgacggc aagagcttcc agcagatggc gggctccatg     300

accggcatga ccgaagaaga gtaccgcgcc atgatgatgg gcggcggccg ctctgccgat     360

ggcaatcgct atgtgggcga ggacggtgat gcgcaggcgc atcgccagcc ccagggcagc 420

gcaggcaacc agaacaagga aggcaactaa 450

<210> 5
<211> 26
<212> DNA
<213> Artificial Sequence

<220>
<223> aF Priemr

<400> 5
atgwssctga tgaarscsga raaccg 26

<210> 6
<211> 27
<212> DNA
<213> Artificial Sequence

<220>
<223> bF Priemr

<400> 6
gtswtcctgg tstayaacga ycaygcy 27

<210> 7
<211> 25
<212> DNA
<213> Artificial Sequence

<220>
<223> bR Priemr

<400> 7
ccctgmarct grtggctcat gccgc 25

<210> 8
<211> 5386
<212> DNA
<213> Comamonas sp. E6

<400> 8
atgatcattg acgtacacgg tcactacacc acggcgcctg cggctctggg cgcatggcgc 60

gatctgcaga tcgccggcct caaggacccg agcaagaccc cgtcggtggc cgatctgaag 120

atcagcgacg atgaaatccg cgagaccatc gaaaccaatc agctgcgcct gatgaaggag 180

cgtggttccg atctgaccat cttcagcccc cgtgcctcgt tcatggcgca ccacatcggt 240

gacttccaga cctccagcac ctgggccgcc atctgcaacg agctgtgctt ccgcgtcagc 300

gagctgttcc ccgaccactt cattcccgcc gccatgctgc cccagtcgcc cggcgtggac 360

cctgcaacct gcattcccga gctggtcaag tgcgttgagc aatatggcaa cgtgggcctg 420

aacctgaacc ccgatccctc gggcggtcac tggacttcgc ctccgctgtc cgacaagagc 480

tggtacccca tctacgaaaa gatggtggag tacgacatcc ccgcgatgat ccacgtctcc 540

accagctgca atagctgctt ccacaccacg ggcagccact atctgaatgc cgacaccacg 600

gccttcatgc agtgcctgac ttcggatctg ttcaaggact tcccgaccct gaagttcctg 660

attccccatg gcggcggcgc cgtgccttac cactggggcc gtttccgcgg tctggcgcag 720

gagatgaaga agccgctgct ggaagagcat ttgctcaaca acatctactt cgacacctgc 780

gtctaccacc agccgggcat caacctgctg acggaagtga ttccgaccaa gaacattctg 840

ttcgccagcg aaatgatcgg cgccgtgcgc ggcatcgatc cgcagaccgg tcactactac 900

gacgacacca agcgctacat cgaagccacg cagaacctga cggccgatga aaagcacgcc 960

gtctacgaag gcaatgcccg ccgcgtgttc acgcgcctgg acaaggcgct caaggccaag 1020

ggtctgtaac ttctaaaaaa gagagctgct ggcgcacggt atgaaaggtt ttcaataaga 1080

aaactattca gatcttatga atagcaagcg ctaacagctc actttttaga ttcaataacg 1140

tcaaagcaaa gaggtatttc catgtacgaa ctgggagttg tctaccgcaa tatccagcgc 1200

gccgaccgcg ctgctgctga cggcctggcc gccctgggct ctgccaccgt gcacgaggcc 1260

atgggccgcg ttggtctgct caagccctat atgcgcccca tctatgccgg caagcaggtc 1320

tcgggtacgg ccgtcacggt gctgctgcag cccggcgaca actggatgat gcatgtggct 1380

gccgagcaga ttcagcccgg cgatatcgtg gtcgctgccg tcaccgccga gtgctccgac 1440

ggctacttcg gcgacctgct ggccaccagc ttccaggcgc gcggcgcacg cgcgctgatc 1500

atcgatgccg gcgtgcgcga cgtgaagacg ctgcaggaga tggacttccc ggtctggagc 1560

aaggccatct cttccaaggg cacgatcaag gccaccctgg gctcggtcaa catccccatc 1620

gtctgcgccg gcatgctggt cacgcccggt gacgtgatcg tggccgacga cgacggcgtg 1680

gtctgcgtgc ccgccgcgcg tgccgtggaa gtgctggccg ctgcccagaa gcgcgaaagc 1740

ttcgaaggcg aaaagcgcgc caagctggct tcgggcgtcc tgggcctgga tatgtacaag 1800

atgcgcgagc ccctggaaaa ggccggcttg cgctatgtgg attaactccc cctgagcggc 1860

tgcgccgctt ccccctctct cgctgcgcgg gaaggggacg acaccctcgg tgcggggcgg 1920

cccttcctcg gtgtctctga gatgtggcag cgccagtttc atggatggtg ggaagtgcgc 1980

agcgcacggt gcaatcaaca attgaggagc aaacagatga gcgcctttga aaaaaccccc 2040

ggctggctgg actggtatgc caaccccagc aagccccagt tcaagctgcc tgccggcgct 2100

gtggatgcgc actgccatgt gttcggtccc ggtaacgagt tccccttcgc ccccgagcgc 2160

aagtacaccc cctgcgacgc cagcaaggcc cagctgtatg cgctgcgcga ccatctgggt 2220

tttgcgcgca atgtggtggt gcaggccacc tgccacggtg cggacaaccg cgccatggtc 2280

gatgcctgca agtcctcggg cggcaaggcc cgcggcgtgg ccacggtcaa gcgctccatc 2340

agcgatgccg aactgcagga gctgcatgac gccggcgtgc gcggcgtgcg tttcaacttc 2400

gtcaagcgtc tggtggactt tacgcccaag gacgagctga tggagatcgc cggtcgcatc 2460

gccaagctgg gctggcatgt ggtgatctat ttcgaagccg tggatctgcc cgagctgtgg 2520

gacttcttca ccgcgctgcc caccaccgtg gtggtcgacc acatgggccg ccccgacgtg 2580

accaagggcg tggacagcga ggagttcgcc ctgttcttga agttcatgcg cgagcacaag 2640

aatgtctgga gcaaggtttc ctgccccgag cgcctgtccg tctccggccc caaggcgctt 2700

catggtgagc agaacgccta ccaggacgtg gtgcctttcg cgcgccgcgt ggtcgaggag 2760

ttccccgagc gcgtgctctg gggcacggac tggccgcacc ccaacctgaa ggaccacatg 2820

cccgacgacg gcctgctggt ggacttcatt cctcatatcg ctcctaccgc gcagctgcag 2880

caaaagctgc tggtggacaa ccccatgcgt ctgtactggc ccgaagaggt ctgacggagt 2940

tccggcgaca catacacccg gaaacaggcc gcagccttgt cgcatgggct gcgccatccg 3000

taacgaggag aatttatggc tttggaaaaa ccgtatctgg acgtgcccgg caccatcatt 3060

ttcgatgccg agcagtcccg caagggctac tggctcaacc agttctgcat gagcctgatg 3120

aaggccgaga accgcgagcg ctttcgcgcc aacgagcgtg cctatctgga cgagtgggcg 3180

atgaccgagg agcagaagca ggccgtgctg gcgcgtgacc tgaactggtg catgcgcacc 3240

ggcggcaata tctacttcct ggccaagatt ggcgccaccg acggcaagag cttccagcag 3300

atggcgggct ccatgaccgg catgaccgaa gaagagtacc gcgccatgat gatgggcggc 3360

ggccgctctg ccgatggcaa tcgctatgtg ggcgaggacg gtgatgcgca ggcgcatcgc 3420

cagccccagg gcagcgcagg caaccagaac aaggaaggca actaagacat ggcacgcatc 3480

accgcatccg ttttcacctc gcacgtgcct gccatcggcg ccgccatgga catgggcaag 3540

acccaggaag cctactgggc gcccctgttc aagggttatg acttctcccg ccagtggatg 3600

aaggacaaca agcccgatgt gatcttcctg gtctacaacg accacgccac ggccttcagc 3660

ctggactgca ttcccacctt cgccatcggc acggctgcgg aattccagcc cgccgacgaa 3720

ggctggggcc cgcgccccgt gcccaaggtg gtcggccatc ccgatctggc cagccacatt 3780

gcccagtccg tgatccagca ggacttcgat ctgaccatcg tcaacaagat ggacgtggac 3840

cacggcctca cggtgcctct gtcgctgatg tgcggcgagc aggaccccaa gaccggctcc 3900

tggccctgcc cggtgatccc cttcgccgtg aacgtggtgc agtatcccgt gcccaccggc 3960

cagcgctgct tcaacctggg ccgcgccatc cgcaaggccg tggagagcta cgaccaggac 4020

atcaacgtcc atatctgggg cacgggtggc atgagccacc agctgcaggg tgcgcgcgca 4080

ggcctgatca acaaggaatg ggacaaccag ttcctggacc tgctgatcga gaacccccac 4140

ggtctggcgc agatgccgca catcgactac gtgcgcgaag ccggctcgga aggcatcgag 4200

ctggtgatgt ggctgattgc gcgcggcgcc atgtccgatg tggacggccc cgcaccgctg 4260

cccaaggtgg cgcaccgctt ctaccatgtg cctgcatcga acaccgcggt gggccatctg 4320

atcctcgaga atcagtaaac gtttcaccac gttcgctgct tcgcgtattc actgccccct 4380

gtggggggct tcgcctcctt gaggcggctc tacggagatc ccaatcatga gcaagaccat 4440

caaagtagcg ctggctggcg caggtgcctt cggcatcaag cacctggacg gcatcaagaa 4500

catcgacggc gtggaagtcg tctccctggt cggtcgccgc tttgaccaga ccaaggaagt 4560

ggccgacaaa tacggcatca agcatgtggc aaccgatctg gccgaaagcc tggcgctgcc 4620

cgaggtcgat gccgtgatcc tttgcacgcc cacgcagatg cacgccgagc aggccattgc 4680

ctgcatgaag gccggcaagc atgtgcaggt cgagattcct ctggccgatg ccctgaagga 4740

cgcccaggaa gtggccgagc tgcaaaagca gaccggtctg gtggccatgg tgggtcacac 4800

ccgccgcttc aaccccagcc accagtgggt gcacaagaag atagcagccg gcgagttcaa 4860

catccagcag atggatgtgc aaacctactt cttccgccgc accaatatga acgcgctggg 4920

ccaggcccgc agctggaccg accacctgct gtggcaccat gccgcccaca ccgtggacct 4980

gttcgcctac caggccggca gccccatcgt caaggccaac gccgtgcaag gcccgattca 5040

caaggatctg ggcatcgcca tggacatgag catccagctc aaggccgcca acggcgcgat 5100

ctgcacgctg agcctgtcgt tcaacaatga cggccctctg ggtaccttct tccgctacat 5160

cggcgacacc ggcacctatc tggcccgcta cgacgatctg tacaccggca aggacgagaa 5220

gatcgacgtg tcccaggtcg atgtgtccat gaacggcatc gagctgcagg accgcgaatt 5280

cttcgccgcc atccgtgaag gccgcgagcc caactccagc gtgcagcagg tgttcaactg 5340

ctacaaggtc ttgcacgacc tggagcagca actcaacgcc gaataa 5386

<210> 9
<211> 768
<212> DNA
<213> Comamonas sp.

<400> 9
atgcaggaca agaactttgt ggaatcgctg cgcaagggat tgggggtact gacttgcttt 60

gaccgtcggc atacccggct gacgctgtca gaggtagcca ggctcacgca gtccacgcca 120

gcatccgcca gacgttcgct cagcacactg gtacagcttg gctatctaga gagcgacggc 180

aaactgttct ggatgcagcc caaatcgctg ctgatcgcct attcatttct gtcatcgcgc 240

cccatgcctg cattggccca gccactactg gatgcactgt cggagcgcac cagagaatcc 300

gcttcgcttg gtactttgtt ggaggacgat gccatcatca ttggtcgttc gaccgcacgg 360

cgcagcttga gcacgggcct aggaatagga tctaggttgc cggtgtactg ctctgcgatt 420

ggtcggatgc tgttgtcagg actcccccaa caggaggcgc gtgcaaggct agagatgatc 480

gagcgggtgg cactgacccc tcatacggtg actgacttgg aggagctgct aggtctgctt 540

gaaacttgcc ggcaatcagg gtggtcatgc agcgacggag agctggagct gggggtgcgc 600

tctatggcag cgccagtgcg cgaccctcaa ggcaacacaa ttgctgccat gagcattgct 660

gttagggcag agagactcag catgagtgag ttcaaggaga cttttttgat accgctgaag 720

cgcgctcgca atgagttgga aaaaaagcta tatccgcagg ggttgtag 768

<210> 10
<211> 969
<212> DNA
<213> Comamonas sp.

<400> 10
atgcgcaacg aatctattcg cagacgcgaa gcattgatcg gcattgccgc tgcggtcgct 60

gcaacaggta gcctggctca gtcaaatcaa cctctcaaga tcgtcgtgcc tttctcagcg 120

ggaggtaccg ccgatgtact gccgcgccta gtagccgaaa aaatccgcgc ggactacgca 180

ggtggcgtga tcattgaaaa caagcccggg gctggtggaa acatcggtgc cgatctagtg 240

ttccgggcac cgccagacgg aatgactgtg ctagcctccc ctccggggcc catcgccatc 300

aaccacaacc tctatcaaaa gctcagcttc gacccgactc gctgggtacc ggtgacgatt 360

ttggccacag tgcccaacgt cctggtcatc aacccgaagc tgcctgttaa gtcacttggc 420

gagttcatcg catacgccaa agccaatccc aaaaaagtga ctgtggcgac gcaaggcgac 480

ggctccacat cccacctgac tgctgccatg tttatgcagc tgactggcac tgaactgact 540

gtcatccctt acaaggaaac agccccggca ctgattgact tgatcggcgg caatgtggat 600

gtgtttttcg acaacatcag ctcgtcggcc acctaccacc aggccggcaa ggtgcgcatt 660

ctggccgttg ccgacgagca gcgctctcaa atattgcccc aggtccccac cttcgccgag 720

cagcaatggc ccgccatgca agccgtgact ttcttttctg ttgtggctcc cccaggcacg 780

agtgcagaaa tcgctcagaa gcttcagaag cagatggccc tggctctgtc atcgaacgac 840

atccgcaagc actttcagga acaaggtgcc gtgccttgtg gttgggaccc gtccaagact 900

gctcagttca tccgccagga aaccgagaag tggaagaagg tgctgaaggc cgccaacgtc 960

aagctctaa 969

<210> 11
<211> 1242
<212> DNA
<213> Comamonas sp.

<400> 11
atgcaagaat ccatcatcca gtggcatggg gccactaata cgcgcgtgcc ttttggtatc 60

tataccgaca cagccaatgc tgatcaggaa cagcagcgca tctatcgcgg cgaggtctgg 120

aactacttgt gcctggaatc tgaaattccc ggggccggtg atttccgcac tacctttgcc 180

ggtgaaacac cgatagttgt cgtacgggat gccgaccagg aaatctacgc cttcgagaac 240

cgctgcgcgc atcgcggcgc tctcatcgct ctggagaaat cgggccgtac ggatagtttc 300

cagtgcgtct atcacgcctg gagctacaac cgacagggag atctgaccgg cgttgccttc 360

gagaaaggtg tcaagggcca gggtggcatg ccggcctcat tctgcaaaga agagcatggc 420

ccgcgcaagc tccgcgtggc tgtcttttgc ggtttggtct ttggcagttt ttccgaggac 480

gtgcccagca ttgaggatta ccttggccct gagatttgcg agcgcataga gcgcgtgctg 540

cacaagcccg tagaagtcat cggtcgcttc acgcaaaagc tgcctaacaa ctggaagctc 600

tacttcgaga acgtgaagga cagctatcac gccagcctcc tgcatatgtt cttcaccacc 660

ttcgagctga atcgcctctc acaaaaaggc ggtgtcatcg tcgacgagtc gggtggccac 720

catgtgagct attccatgat cgatcgcggc gccaaagacg actcgtacaa ggaccaggcc 780

atccgctccg acaacgagcg ttaccggctc aaagatccta gccttctaga gggctttgag 840

gagttcgagg acggcgtgac cctgcagatc ctttctgtgt tccctggctt tgtgctgcag 900

cagattcaga acagcatcgc cgtgcgtcag ttgctgccca agagcatctc cagctcggaa 960

ctcaactgga cctatcttgg ctatgcagat gacagtgccg agcaacgcaa ggtcagactc 1020

aaacaggcca accttatcgg cccggccgga ttcatttcca tggaagacgg agctgtcggt 1080

ggattcgtgc agcgtggcat cgcaggcgct gccaaccttg atgcagtcat cgagatgggc 1140

ggagaccacg aaggctctag cgagggccgc gccacggaaa cctcggtacg cggcttttgg 1200

aaggcctacc gcaagcatat gggacaggag atgcaagcat ga 1242

<210> 12
<211> 465
<212> DNA
<213> Comamonas sp.

<400> 12
atgatcaatg aaattcaaat cgcggccttc aatgccgcct acgcgaagac catagacagt 60

gatgcaatgg agcaatggcc aacctttttc accaaggatt gccactattg cgtcaccaat 120

gtcgacaacc atgatgaggg acttgctgcc ggcattgtct gggcggattc gcaggacatg 180

ctcaccgacc gaatttctgc gctgcgcgaa gccaatatct acgagcgcca ccgctatcgc 240

catatcctgg gtctgccttc gatccagtca ggcgatgcaa cacaggccag cgcttccact 300

ccgttcatgg tgctgcgcat catgcataca ggggaaacag aggtctttgc cagcggtgag 360

tacctcgaca aattcaccac gatcgatggc aagttacgtc tgcaagaacg catcgcggtt 420

tgcgacagca cggtgacgga cacgctgatg gcattgccgc tatga 465

<210> 13
<211> 948
<212> DNA
<213> Comamonas sp.

<400> 13
atgacaatag tgcaccgtag attggctttg gccatcggcg atccccacgg tattggccca 60

gaaatcgcac tgaaagctct ccagcagctg tctgtcaccg aaaggtctct tatcaaggtc 120

tatggacctt ggagcgctct cgagcaagcc gcacgggttt gcgaaatgga gccgcttctt 180

caagacatcg ttcacgagga agccggcaca cttacacaac cagttcaatg gggagaaatc 240

accccgcagg ctggtctatc tacggtgcaa tccgcaacag cggctatccg agcgtgcgaa 300

aacggcgaag tcgatgccgt cattgcctgc cctcaccatg aaacggccat tcaccgcgca 360

ggcatagcgt tcagcggcta cccatctttg ctcgccaatg ttcttggcat gaacgaagac 420

caggtattcc tgatgctggt gggggctggc ctgcgcatag tgcatgtcac tttgcatgaa 480

agcgtgcgca gcgcattgga gcggctctct cctcagttgg tggtcaacgc ggcgcaggct 540

gccgtgcaga catgcacctt actcggagtg cctaaaccaa aagtcgctgt attcgggatc 600

aaccctcatg catctgaagg acagttgttc ggcctggagg actcccagat caccgttccc 660

gctgtcgaga cactgcgcaa gcgcggccta gcagtagacg gccccatggg agctgacatg 720

gttctggcac agcgcaagca cgacctgtat gtagccatgc tgcacgatca gggccatatc 780

cccatcaagc tgctggcacc taacggagcc agcgcactat ctatcggtgg cagggtggtg 840

ctttccagcg tgggccatgg cagcgccatg gacattgccg gccgtggcgt ggctgacgcc 900

acggccctcc tacgcacaat agccctactc ggagcccaac cggtctga 948

<210> 14
<211> 1011
<212> DNA
<213> Comamonas sp.

<400> 14
atgaaccacc agatccatat ccacgactcc gatatcgcgt tcccctgcgc gcccgggcaa 60

tccgtactgg atgcagctct gcaggccggc atcgagctgc cctattcctg ccgcaaaggt 120

agctgtggca actgtgcgag tacgctgctc gacggaaata ttgcctcctt caatggcatg 180

gccgtgcgaa acgaactctg cgcctcggaa caagtgctgc tgtgcggctg cactgcagcc 240

agcgatatac gtatccaccc gagctccttt cgccgtctcg acccggaagc ccgaaaacgt 300

tttacggcca aggtgtacag caatacactg gcggcacccg atgtctcgct gctgcgcctg 360

cgcctgcctg tgggcaagcg cgccaaattt gaagccggcc aatacctgct gattcacctc 420

gacgacgggg aaagccgcag ctactctatg gccaatccac cccatgagag cgatggcatc 480

acattgcatg tcaggcatgt acctggtggt cgcttcagca ctatcgttca gcagttgaag 540

tctggtgaca cattggatat cgaactgcca ttcggcagca tcgcactgaa gcctgatgac 600

gcaaggcccc tgatttgcgt tgcgggtggc acgggatttg cgcccattaa atccgttctt 660

gatgacttag ccaaacgcaa ggttcagcgc gacatcacgc tgatctgggg ggctcgcaac 720

ccctcgggcc tgtatcttcc tagcgccatc gacaagtggc gcaaagtctg gccacagttt 780

cgctacattg cagccatcac cgacctaggc gatatgcctg cggatgctca cgcaggtcgg 840

gtggatgacg cgctacgcac tcactttggc aacctgcacg atcatgtggt gcactgctgt 900

ggctcaccag ctctggttca atcagtgcgc acagccgctt ccgatatggg cctgcttgca 960

caggacttcc acgcggatgt ttttgcgaca ggcccgactg gtcaccacta g 1011

<210> 15
<211> 720
<212> DNA
<213> Pseudomonas putida KT2440

<400> 15
atgcccgccc aggacaacag ccgcttcgtg atccgtgatc gcaactggca ccctaaagcc 60

cttacgcctg actacaagac ctccgttgcc cgctcgccgc gccaggcact ggtcagcatt 120

ccgcagtcga tcagcgaaac cactggtccg gacttttccc atctgggctt cggcgcccac 180

gaccatgacc tgctgctgaa cttcaataac ggtggcctgc ccattggcga gcgcatcatc 240

gtcgccggcc gtgtcgtcga ccagtacggc aagcctgtgc cgaacacttt ggtggagatg 300

tggcaagcca acgccggcgg ccgctatcgc cacaagaacg atcgctacct ggcgcccctg 360

gacccgaact tcggtggtgt tgggcggtgt ctgaccgacc gtgacggcta ttacagcttc 420

cgcaccatca agccgggccc gtacccatgg cgcaacggcc cgaacgactg gcgcccggcg 480

catatccact tcgccatcag cggcccatcg atcgccacca agctgatcac ccagttgtac 540

ttcgaaggtg acccgctgat cccgatgtgc ccgatcgtca agtcgatcgc caacccgcaa 600

gccgtgcagc agttgatcgc caagctcgac atgagcaacg ccaacccgat ggactgcctg 660

gcctaccgct ttgacatcgt gctgcgcggc cagcgcaaga cccacttcga aaactgctga 720

<210> 16
<211> 606
<212> DNA
<213> Pseudomonas putida KT2440

<400> 16
   atgccaatcg aactgctgcc ggaaacccct tcgcagactg ccggccccta cgtgcacatc 60
   ggcctggccc tggaagccgc cggcaacccg acccgcgacc aggaaatctg gaactgcctg 120
   gccaagccag acgccccggg cgagcacatt ctgctgatcg gccacgtata tgacggaaac 180
   ggccacctgg tgcgcgactc gttcctggaa gtgtggcagg ccgacgccaa cggtgagtac 240
   caggatgcct acaacctgga aaacgccttc aacagctttg gccgcacggc taccaccttc 300
   gatgccggtg agtggacgct gcaaacggtc aagccgggtg tggtgaacaa cgctgctggc 360
   gtgccgatgg cgccgcacat caacatcagc ctgtttgccc gtggcatcaa catccacctg 420
   cacacgcgcc tgtatttcga tgatgaggcc caggccaatg ccaagtgccc ggtgctcaac 480
   ctgatcgagc agccgcagcg gcgtgaaacc ttgattgcca agcgttgcga agtggatggg 540
   aagacggcgt accgctttga tatccgcatt cagggggaag gggagaccgt cttcttcgac 600
   ttctga 606

SEQUENCE LISTING

<110>  TOYOTA INDUSTRIES CORPORATION/ Tokyo University of Agriculture and
       Technology/ Nagaoka University of Technology/ Forestry and Forest
       Products Research Institute

<120>  Mutant, Recombinat Plasmid, Transformant and Method for
       Producing 3-Carboxymuconolactone

<130>  V576-PCT

<160>  16

<170>  PatentIn version 3.4

<210>  1
<211>  814
<212>  PRT
<213>  Comamonas sp.E6

<400>  1

Met Glu Thr Ala Leu Ala Ala Arg Gly Ile Leu Glu Thr His Arg Ala
1               5                   10                  15

Leu Ala Ser Glu Arg Val Ala Leu Pro His Glu Thr His Arg Ser Glu
            20                  25                  30

Arg His Ile Ser Val Ala Leu Pro Arg Ala Leu Ala Ile Leu Glu Gly
        35                  40                  45

Leu Tyr Ala Leu Ala Ala Leu Ala Met Glu Thr Ala Ser Pro Met Glu
    50                  55                  60

Thr Gly Leu Tyr Leu Tyr Ser Thr His Arg Gly Leu Asn Gly Leu Ala
65                  70                  75                  80

Leu Ala Thr Tyr Arg Thr Arg Pro Ala Leu Ala Pro Arg Leu Glu Pro
                85                  90                  95

His Glu Leu Tyr Ser Gly Leu Tyr Thr Tyr Arg Ala Ser Pro Pro His
            100                 105                 110

Glu Ser Glu Arg Ala Arg Gly Gly Leu Asn Thr Arg Pro Met Glu Thr
        115                 120                 125

Leu Tyr Ser Ala Ser Pro Ala Ser Asn Leu Tyr Ser Pro Arg Ala Ser
    130                 135                 140

Pro Val Ala Leu Ile Leu Glu Pro His Glu Leu Glu Val Ala Leu Thr
145                 150                 155                 160

Tyr Arg Ala Ser Asn Ala Ser Pro His Ile Ser Ala Leu Ala Thr His
                165                 170                 175

Arg Ala Leu Ala Pro His Glu Ser Glu Arg Leu Glu Ala Ser Pro Cys
            180                 185                 190

Tyr Ser Ile Leu Glu Pro Arg Thr His Arg Pro His Glu Ala Leu Ala
        195                 200                 205

Ile Leu Glu Gly Leu Tyr Thr His Arg Ala Leu Ala Ala Leu Ala Gly
    210                 215                 220

Leu Pro His Glu Gly Leu Asn Pro Arg Ala Leu Ala Ala Ser Pro Gly
225                 230                 235                 240

Leu Gly Leu Tyr Thr Arg Pro Gly Leu Tyr Pro Arg Ala Arg Gly Pro
                245                 250                 255

Arg Val Ala Leu Pro Arg Leu Tyr Ser Val Ala Leu Val Ala Leu Gly
            260                 265                 270

Leu Tyr His Ile Ser Pro Arg Ala Ser Pro Leu Glu Ala Leu Ala Ser
        275                 280                 285

Glu Arg His Ile Ser Ile Leu Glu Ala Leu Ala Gly Leu Asn Ser Glu
    290                 295                 300

Arg Val Ala Leu Ile Leu Glu Gly Leu Asn Gly Leu Asn Ala Ser Pro
305                 310                 315                 320

Pro His Glu Ala Ser Pro Leu Glu Thr His Arg Ile Leu Glu Val Ala
                325                 330                 335

Leu Ala Ser Asn Leu Tyr Ser Met Glu Thr Ala Ser Pro Val Ala Leu
            340                 345                 350

Ala Ser Pro His Ile Ser Gly Leu Tyr Leu Glu Thr His Arg Val Ala
        355                 360                 365

Leu Pro Arg Leu Glu Ser Glu Arg Leu Glu Met Glu Thr Cys Tyr Ser
    370                 375                 380

Gly Leu Tyr Gly Leu Gly Leu Asn Ala Ser Pro Pro Arg Leu Tyr Ser
385                 390                 395                 400

Thr His Arg Gly Leu Tyr Ser Glu Arg Thr Arg Pro Pro Arg Cys Tyr
                405                 410                 415

Ser Pro Arg Val Ala Leu Ile Leu Glu Pro Arg Pro His Glu Ala Leu
            420                 425                 430

Ala Val Ala Leu Ala Ser Asn Val Ala Leu Val Ala Leu Gly Leu Asn
        435                 440                 445

Thr Tyr Arg Pro Arg Val Ala Leu Pro Arg Thr His Arg Gly Leu Tyr
    450                 455                 460

Gly Leu Asn Ala Arg Gly Cys Tyr Ser Pro His Glu Ala Ser Asn Leu
465                 470                 475                 480

Glu Gly Leu Tyr Ala Arg Gly Ala Leu Ala Ile Leu Glu Ala Arg Gly
                485                 490                 495

Leu Tyr Ser Ala Leu Ala Val Ala Leu Gly Leu Ser Glu Arg Thr Tyr
            500                 505                 510

Arg Ala Ser Pro Gly Leu Asn Ala Ser Pro Ile Leu Glu Ala Ser Asn
        515                 520                 525

Val Ala Leu His Ile Ser Ile Leu Glu Thr Arg Pro Gly Leu Tyr Thr
    530                 535                 540

His Arg Gly Leu Tyr Gly Leu Tyr Met Glu Thr Ser Glu Arg His Ile
545                 550                 555                 560

Ser Gly Leu Asn Leu Glu Gly Leu Asn Gly Leu Tyr Ala Leu Ala Ala
                565                 570                 575

Arg Gly Ala Leu Ala Gly Leu Tyr Leu Glu Ile Leu Glu Ala Ser Asn
            580                 585                 590

Leu Tyr Ser Gly Leu Thr Arg Pro Ala Ser Pro Ala Ser Asn Gly Leu
        595                 600                 605

Asn Pro His Glu Leu Glu Ala Ser Pro Leu Glu Leu Glu Ile Leu Glu
    610                 615                 620

Gly Leu Ala Ser Asn Pro Arg His Ile Ser Gly Leu Tyr Leu Glu Ala
625                 630                 635                 640

Leu Ala Gly Leu Asn Met Glu Thr Pro Arg His Ile Ser Ile Leu Glu
                645                 650                 655

Ala Ser Pro Thr Tyr Arg Val Ala Leu Ala Arg Gly Gly Leu Ala Leu
            660                 665                 670

Ala Gly Leu Tyr Ser Glu Arg Gly Leu Gly Leu Tyr Ile Leu Glu Gly
        675                 680                 685

Leu Leu Glu Val Ala Leu Met Glu Thr Thr Arg Pro Leu Glu Ile Leu
    690                 695                 700

Glu Ala Leu Ala Ala Arg Gly Gly Leu Tyr Ala Leu Ala Met Glu Thr
705                 710                 715                 720

Ser Glu Arg Ala Ser Pro Val Ala Leu Ala Ser Pro Gly Leu Tyr Pro
                725                 730                 735

Arg Ala Leu Ala Pro Arg Leu Glu Pro Arg Leu Tyr Ser Val Ala Leu
            740                 745                 750

Ala Leu Ala His Ile Ser Ala Arg Gly Pro His Glu Thr Tyr Arg His
        755                 760                 765

Ile Ser Val Ala Leu Pro Arg Ala Leu Ala Ser Glu Arg Ala Ser Asn
    770                 775                 780

Thr His Arg Ala Leu Ala Val Ala Leu Gly Leu Tyr His Ile Ser Leu
785                 790                 795                 800

Glu Ile Leu Glu Leu Glu Gly Leu Ala Ser Asn Gly Leu Asn
                805                 810

<210> 2
<211> 870
<212> DNA
<213> Comamonas sp. E6

<400> 2
atggcacgca tcaccgcatc cgttttcacc tcgcacgtgc ctgccatcgg cgccgccatg      60

gacatgggca agacccagga agcctactgg gcgcccctgt tcaagggtta tgacttctcc     120

cgccagtgga tgaaggacaa caagcccgat gtgatcttcc tggtctacaa cgaccacgcc     180

acggccttca gcctggactg cattcccacc ttcgccatcg gcacggctgc ggaattccag     240

cccgccgacg aaggctgggg cccgcgcccc gtgcccaagg tggtcggcca tcccgatctg     300

gccagccaca ttgcccagtc cgtgatccag caggacttcg atctgaccat cgtcaacaag     360

atggacgtgg accacggcct cacggtgcct ctgtcgctga tgtgcggcga gcaggacccc     420

aagaccggct cctggccctg cccggtgatc cccttcgccg tgaacgtggt gcagtatccc     480

gtgcccaccg gccagcgctg cttcaacctg ggccgcgcca tccgcaaggc cgtggagagc     540

tacgaccagg acatcaacgt ccatatctgg ggcacgggtg gcatgagcca ccagctgcag     600

ggtgcgcgcg caggcctgat caacaaggaa tgggacaacc agttcctgga cctgctgatc 660

gagaaccccc acggtctggc gcagatgccg cacatcgact acgtgcgcga agccggctcg 720

gaaggcatcg agctggtgat gtggctgatt gcgcgcggcg ccatgtccga tgtggacggc 780

cccgcaccgc tgcccaaggt ggcgcaccgc ttctaccatg tgcctgcatc gaacaccgcg 840

gtgggccatc tgatcctcga gaatcagtaa 870

<210> 3
<211> 425
<212> PRT
<213> Comamonas sp. E6

<400> 3

Met Glu Thr Ala Leu Ala Leu Glu Gly Leu Leu Tyr Ser Pro Arg Thr
1               5                   10                  15

Tyr Arg Leu Glu Ala Ser Pro Val Ala Leu Pro Arg Gly Leu Tyr Thr
            20                  25                  30

His Arg Ile Leu Glu Ile Leu Glu Pro His Glu Ala Ser Pro Ala Leu
        35                  40                  45

Ala Gly Leu Gly Leu Asn Ser Glu Arg Ala Arg Gly Leu Tyr Ser Gly
    50                  55                  60

Leu Tyr Thr Tyr Arg Thr Arg Pro Leu Glu Ala Ser Asn Gly Leu Asn
65                  70                  75                  80

Pro His Glu Cys Tyr Ser Met Glu Thr Ser Glu Arg Leu Glu Met Glu
                85                  90                  95

Thr Leu Tyr Ser Ala Leu Ala Gly Leu Ala Ser Asn Ala Arg Gly Gly
            100                 105                 110

Leu Ala Arg Gly Pro His Glu Ala Arg Gly Ala Leu Ala Ala Ser Asn
        115                 120                 125

Gly Leu Ala Arg Gly Ala Leu Ala Thr Tyr Arg Leu Glu Ser Glu Arg
    130                 135                 140

Gly Leu Thr Arg Pro Ala Leu Ala Met Glu Thr Ala Arg Gly Gly Leu
145                 150                 155                 160

Gly Leu Gly Leu Asn Leu Tyr Ser Gly Leu Asn Ala Leu Ala Val Ala
                165                 170                 175

Leu Leu Glu Ala Leu Ala Ala Arg Gly Ala Ser Pro Leu Glu Ala Ser
            180                 185                 190

Asn Thr Arg Pro Cys Tyr Ser Met Glu Thr Ala Arg Gly Thr His Arg
        195                 200                 205

Gly Leu Tyr Gly Leu Tyr Ala Ser Asn Ile Leu Glu Thr Tyr Arg Pro
    210                 215                 220

His Glu Leu Glu Ala Leu Ala Leu Tyr Ser Ile Leu Glu Gly Leu Tyr
225                 230                 235                 240

Ala Leu Ala Thr His Arg Ala Ser Pro Gly Leu Tyr Leu Tyr Ser Ser
                245                 250                 255

Glu Arg Pro His Glu Gly Leu Asn Gly Leu Asn Met Glu Thr Ala Leu
            260                 265                 270

Ala Gly Leu Tyr Ser Glu Arg Met Glu Thr Thr His Arg Gly Leu Tyr
        275                 280                 285

Met Glu Thr Thr His Arg Gly Leu Gly Leu Gly Leu Thr Tyr Arg Ala
    290                 295                 300

Arg Gly Ala Leu Ala Met Glu Thr Met Glu Thr Met Glu Thr Gly Leu
305                 310                 315                 320

Tyr Gly Leu Tyr Gly Leu Tyr Ala Arg Gly Ser Glu Arg Ala Leu Ala
                325                 330                 335

Ala Ser Pro Gly Leu Tyr Ala Ser Asn Ala Arg Gly Thr Tyr Arg Val
            340                 345                 350

Ala Leu Gly Leu Tyr Gly Leu Ala Ser Pro Gly Leu Tyr Ala Ser Pro
        355                 360                 365

Ala Leu Ala Gly Leu Asn Ala Leu Ala Ile Leu Glu Ile Leu Glu Ala
    370                 375                 380

Arg Gly Gly Leu Asn Pro Arg Gly Leu Asn Gly Leu Tyr Ser Glu Arg
385                 390                 395                 400

Ala Leu Ala Gly Leu Tyr Ala Ser Asn Gly Leu Asn Ala Ser Asn Gly
                405                 410                 415

Leu Gly Leu Gly Leu Tyr Ala Ser Asn
            420                 425

<210> 4
<211> 450
<212> DNA
<213> Comamonas sp. E6

<400> 4
atggctttgg aaaaaccgta tctggacgtg cccggcacca tcattttcga tgccgagcag      60

tcccgcaagg gctactggct caaccagttc tgcatgagcc tgatgaaggc cgagaaccgc     120

gagcgctttc gcgccaacga gcgtgcctat ctggacgagt gggcgatgac cgaggagcag     180

aagcaggccg tgctggcgcg tgacctgaac tggtgcatgc gcaccggcgg caatatctac     240

ttcctggcca agattggcgc caccgacggc aagagcttcc agcagatggc gggctccatg     300

accggcatga ccgaagaaga gtaccgcgcc atgatgatgg gcggcggccg ctctgccgat     360

ggcaatcgct atgtgggcga ggacggtgat gcgcaggcgc atcgccagcc ccagggcagc 420

gcaggcaacc agaacaagga aggcaactaa 450

<210> 5
<211> 26
<212> DNA
<213> Artificial Sequence

<220>
<223> aF Priemr

<400> 5
atgwssctga tgaarscsga raaccg 26

<210> 6
<211> 27
<212> DNA
<213> Artificial Sequence

<220>
<223> bF Priemr

<400> 6
gtswtcctgg tstayaacga ycaygcy 27

<210> 7
<211> 25
<212> DNA
<213> Artificial Sequence

<220>
<223> bR Priemr

<400> 7
ccctgmarct grtggctcat gccgc 25

<210> 8
<211> 5386
<212> DNA
<213> Comamonas sp. E6

<400> 8
atgatcattg acgtacacgg tcactacacc acggcgcctg cggctctggg cgcatggcgc 60

gatctgcaga tcgccggcct caaggacccg agcaagaccc cgtcggtggc cgatctgaag 120

atcagcgacg atgaaatccg cgagaccatc gaaaccaatc agctgcgcct gatgaaggag 180

cgtggttccg atctgaccat cttcagcccc cgtgcctcgt tcatggcgca ccacatcggt 240

gacttccaga cctccagcac ctgggccgcc atctgcaacg agctgtgctt ccgcgtcagc 300

gagctgttcc ccgaccactt cattcccgcc gccatgctgc cccagtcgcc cggcgtggac 360

cctgcaacct gcattcccga gctggtcaag tgcgttgagc aatatggcaa cgtgggcctg 420

aacctgaacc ccgatccctc gggcggtcac tggacttcgc ctccgctgtc cgacaagagc 480

tggtacccca tctacgaaaa gatggtggag tacgacatcc ccgcgatgat ccacgtctcc 540

accagctgca atagctgctt ccacaccacg ggcagccact atctgaatgc cgacaccacg 600

gccttcatgc agtgcctgac ttcggatctg ttcaaggact tcccgaccct gaagttcctg 660

attccccatg gcggcggcgc cgtgccttac cactggggcc gtttccgcgg tctggcgcag 720

gagatgaaga agccgctgct ggaagagcat ttgctcaaca acatctactt cgacacctgc 780

gtctaccacc agccgggcat caacctgctg acggaagtga ttccgaccaa gaacattctg 840

ttcgccagcg aaatgatcgg cgccgtgcgc ggcatcgatc cgcagaccgg tcactactac 900

gacgacacca agcgctacat cgaagccacg cagaacctga cggccgatga aaagcacgcc 960

gtctacgaag gcaatgcccg ccgcgtgttc acgcgcctgg acaaggcgct caaggccaag 1020

ggtctgtaac ttctaaaaaa gagagctgct ggcgcacggt atgaaaggtt ttcaataaga 1080

aaactattca gatcttatga atagcaagcg ctaacagctc actttttaga ttcaataacg 1140

tcaaagcaaa gaggtatttc catgtacgaa ctgggagttg tctaccgcaa tatccagcgc 1200

gccgaccgcg ctgctgctga cggcctggcc gccctgggct ctgccaccgt gcacgaggcc 1260

atgggccgcg ttggtctgct caagccctat atgcgcccca tctatgccgg caagcaggtc 1320

tcgggtacgg ccgtcacggt gctgctgcag cccggcgaca actggatgat gcatgtggct 1380

gccgagcaga ttcagcccgg cgatatcgtg gtcgctgccg tcaccgccga gtgctccgac 1440

ggctacttcg gcgacctgct ggccaccagc ttccaggcgc gcggcgcacg cgcgctgatc 1500

atcgatgccg gcgtgcgcga cgtgaagacg ctgcaggaga tggacttccc ggtctggagc 1560

aaggccatct cttccaaggg cacgatcaag gccaccctgg gctcggtcaa catccccatc 1620

gtctgcgccg gcatgctggt cacgcccggt gacgtgatcg tggccgacga cgacggcgtg 1680

gtctgcgtgc ccgccgcgcg tgccgtggaa gtgctggccg ctgcccagaa gcgcgaaagc 1740

ttcgaaggcg aaaagcgcgc caagctggct tcgggcgtcc tgggcctgga tatgtacaag 1800

atgcgcgagc ccctggaaaa ggccggcttg cgctatgtgg attaactccc cctgagcggc 1860

tgcgccgctt ccccctctct cgctgcgcgg gaaggggacg acaccctcgg tgcggggcgg 1920

cccttcctcg gtgtctctga gatgtggcag cgccagtttc atggatggtg ggaagtgcgc 1980

agcgcacggt gcaatcaaca attgaggagc aaacagatga gcgcctttga aaaaaccccc 2040

ggctggctgg actggtatgc caaccccagc aagccccagt tcaagctgcc tgccggcgct 2100

gtggatgcgc actgccatgt gttcggtccc ggtaacgagt tccccttcgc ccccgagcgc 2160

aagtacaccc cctgcgacgc cagcaaggcc cagctgtatg cgctgcgcga ccatctgggt 2220

tttgcgcgca atgtggtggt gcaggccacc tgccacggtg cggacaaccg cgccatggtc 2280

gatgcctgca agtcctcggg cggcaaggcc cgcggcgtgg ccacggtcaa gcgctccatc 2340

agcgatgccg aactgcagga gctgcatgac gccggcgtgc gcggcgtgcg tttcaacttc 2400

gtcaagcgtc tggtggactt tacgcccaag gacgagctga tggagatcgc cggtcgcatc 2460

gccaagctgg gctggcatgt ggtgatctat ttcgaagccg tggatctgcc cgagctgtgg 2520

gacttcttca ccgcgctgcc caccaccgtg gtggtcgacc acatgggccg ccccgacgtg 2580

accaagggcg tggacagcga ggagttcgcc ctgttcttga agttcatgcg cgagcacaag 2640

aatgtctgga gcaaggtttc ctgccccgag cgcctgtccg tctccggccc caaggcgctt 2700

catggtgagc agaacgccta ccaggacgtg gtgcctttcg cgcgccgcgt ggtcgaggag 2760

ttccccgagc gcgtgctctg gggcacggac tggccgcacc ccaacctgaa ggaccacatg 2820

cccgacgacg gcctgctggt ggacttcatt cctcatatcg ctcctaccgc gcagctgcag 2880

caaaagctgc tggtggacaa ccccatgcgt ctgtactggc ccgaagaggt ctgacggagt 2940

tccggcgaca catacacccg gaaacaggcc gcagccttgt cgcatgggct gcgccatccg 3000

taacgaggag aatttatggc tttggaaaaa ccgtatctgg acgtgcccgg caccatcatt 3060

ttcgatgccg agcagtcccg caagggctac tggctcaacc agttctgcat gagcctgatg 3120

aaggccgaga accgcgagcg ctttcgcgcc aacgagcgtg cctatctgga cgagtgggcg 3180

atgaccgagg agcagaagca ggccgtgctg gcgcgtgacc tgaactggtg catgcgcacc 3240

ggcggcaata tctacttcct ggccaagatt ggcgccaccg acggcaagag cttccagcag 3300

atggcgggct ccatgaccgg catgaccgaa gaagagtacc gcgccatgat gatgggcggc 3360

ggccgctctg ccgatggcaa tcgctatgtg ggcgaggacg gtgatgcgca ggcgcatcgc 3420

cagccccagg gcagcgcagg caaccagaac aaggaaggca actaagacat ggcacgcatc 3480

accgcatccg ttttcacctc gcacgtgcct gccatcggcg ccgccatgga catgggcaag 3540

acccaggaag cctactgggc gcccctgttc aagggttatg acttctcccg ccagtggatg 3600

aaggacaaca agcccgatgt gatcttcctg gtctacaacg accacgccac ggccttcagc 3660

ctggactgca ttcccacctt cgccatcggc acggctgcgg aattccagcc cgccgacgaa 3720

ggctggggcc cgcgccccgt gcccaaggtg gtcggccatc ccgatctggc cagccacatt 3780

gcccagtccg tgatccagca ggacttcgat ctgaccatcg tcaacaagat ggacgtggac 3840

cacggcctca cggtgcctct gtcgctgatg tgcggcgagc aggaccccaa gaccggctcc 3900

tggccctgcc cggtgatccc cttcgccgtg aacgtggtgc agtatcccgt gcccaccggc 3960

cagcgctgct tcaacctggg ccgcgccatc cgcaaggccg tggagagcta cgaccaggac 4020

atcaacgtcc atatctgggg cacgggtggc atgagccacc agctgcaggg tgcgcgcgca 4080

ggcctgatca acaaggaatg ggacaaccag ttcctggacc tgctgatcga gaacccccac 4140

ggtctggcgc agatgccgca catcgactac gtgcgcgaag ccggctcgga aggcatcgag 4200

ctggtgatgt ggctgattgc gcgcggcgcc atgtccgatg tggacggccc cgcaccgctg 4260

cccaaggtgg cgcaccgctt ctaccatgtg cctgcatcga acaccgcggt gggccatctg 4320

atcctcgaga atcagtaaac gtttcaccac gttcgctgct tcgcgtattc actgccccct 4380

gtggggggct tcgcctcctt gaggcggctc tacggagatc ccaatcatga gcaagaccat 4440

caaagtagcg ctggctggcg caggtgcctt cggcatcaag cacctggacg gcatcaagaa 4500

catcgacggc gtggaagtcg tctccctggt cggtcgccgc tttgaccaga ccaaggaagt 4560

ggccgacaaa tacggcatca agcatgtggc aaccgatctg gccgaaagcc tggcgctgcc 4620

cgaggtcgat gccgtgatcc tttgcacgcc cacgcagatg cacgccgagc aggccattgc 4680

ctgcatgaag gccggcaagc atgtgcaggt cgagattcct ctggccgatg ccctgaagga 4740

cgcccaggaa gtggccgagc tgcaaaagca gaccggtctg gtggccatgg tgggtcacac 4800

ccgccgcttc aaccccagcc accagtgggt gcacaagaag atagcagccg gcgagttcaa 4860

catccagcag atggatgtgc aaacctactt cttccgccgc accaatatga acgcgctggg 4920

ccaggcccgc agctggaccg accacctgct gtggcaccat gccgcccaca ccgtggacct 4980

gttcgcctac caggccggca gccccatcgt caaggccaac gccgtgcaag gcccgattca 5040

caaggatctg ggcatcgcca tggacatgag catccagctc aaggccgcca acggcgcgat 5100

ctgcacgctg agcctgtcgt tcaacaatga cggccctctg ggtaccttct tccgctacat 5160

cggcgacacc ggcacctatc tggcccgcta cgacgatctg tacaccggca aggacgagaa 5220

gatcgacgtg tcccaggtcg atgtgtccat gaacggcatc gagctgcagg accgcgaatt 5280

cttcgccgcc atccgtgaag gccgcgagcc caactccagc gtgcagcagg tgttcaactg 5340

ctacaaggtc ttgcacgacc tggagcagca actcaacgcc gaataa 5386

<210> 9
<211> 768
<212> DNA
<213> Comamonas sp.

<400> 9
atgcaggaca agaactttgt ggaatcgctg cgcaagggat tgggggtact gacttgcttt 60

gaccgtcggc atacccggct gacgctgtca gaggtagcca ggctcacgca gtccacgcca 120

gcatccgcca gacgttcgct cagcacactg gtacagcttg gctatctaga gagcgacggc 180

aaactgttct ggatgcagcc caaatcgctg ctgatcgcct attcatttct gtcatcgcgc 240

cccatgcctg cattggccca gccactactg gatgcactgt cggagcgcac cagagaatcc 300

gcttcgcttg gtactttgtt ggaggacgat gccatcatca ttggtcgttc gaccgcacgg 360

cgcagcttga gcacgggcct aggaatagga tctaggttgc cggtgtactg ctctgcgatt 420

ggtcggatgc tgttgtcagg actcccccaa caggaggcgc gtgcaaggct agagatgatc 480

gagcgggtgg cactgacccc tcatacggtg actgacttgg aggagctgct aggtctgctt 540

gaaacttgcc ggcaatcagg gtggtcatgc agcgacggag agctggagct gggggtgcgc 600

tctatggcag cgccagtgcg cgaccctcaa ggcaacacaa ttgctgccat gagcattgct 660

gttagggcag agagactcag catgagtgag ttcaaggaga ctttttgat accgctgaag 720

cgcgctcgca atgagttgga aaaaaagcta tatccgcagg ggttgtag 768

<210> 10
<211> 969
<212> DNA
<213> Comamonas sp.

<400> 10
atgcgcaacg aatctattcg cagacgcgaa gcattgatcg gcattgccgc tgcggtcgct 60

gcaacaggta gcctggctca gtcaaatcaa cctctcaaga tcgtcgtgcc tttctcagcg 120

ggaggtaccg ccgatgtact gccgcgccta gtagccgaaa aaatccgcgc ggactacgca 180

ggtggcgtga tcattgaaaa caagcccggg gctggtggaa acatcggtgc cgatctagtg 240

ttccgggcac cgccagacgg aatgactgtg ctagcctccc ctccggggcc catcgccatc 300

aaccacaacc tctatcaaaa gctcagcttc gacccgactc gctgggtacc ggtgacgatt 360

ttggccacag tgcccaacgt cctggtcatc aacccgaagc tgcctgttaa gtcacttggc 420

gagttcatcg catacgccaa agccaatccc aaaaaagtga ctgtggcgac gcaaggcgac 480

ggctccacat cccacctgac tgctgccatg tttatgcagc tgactggcac tgaactgact 540

gtcatccctt acaaggaaac agccccggca ctgattgact tgatcggcgg caatgtggat 600

gtgtttttcg acaacatcag ctcgtcggcc acctaccacc aggccggcaa ggtgcgcatt 660

ctggccgttg ccgacgagca gcgctctcaa atattgcccc aggtccccac cttcgccgag 720

cagcaatggc ccgccatgca agccgtgact ttcttttctg ttgtggctcc cccaggcacg 780

agtgcagaaa tcgctcagaa gcttcagaag cagatggccc tggctctgtc atcgaacgac 840

atccgcaagc actttcagga acaaggtgcc gtgccttgtg gttgggaccc gtccaagact 900

gctcagttca tccgccagga aaccgagaag tggaagaagg tgctgaaggc cgccaacgtc 960

aagctctaa 969

<210> 11
<211> 1242
<212> DNA
<213> Comamonas sp.

<400> 11
atgcaagaat ccatcatcca gtggcatggg gccactaata cgcgcgtgcc ttttggtatc 60

tataccgaca cagccaatgc tgatcaggaa cagcagcgca tctatcgcgg cgaggtctgg 120

aactacttgt gcctggaatc tgaaattccc ggggccggtg atttccgcac tacctttgcc 180

ggtgaaacac cgatagttgt cgtacgggat gccgaccagg aaatctacgc cttcgagaac 240

cgctgcgcgc atcgcggcgc tctcatcgct ctggagaaat cgggccgtac ggatagtttc 300

cagtgcgtct atcacgcctg gagctacaac cgacagggag atctgaccgg cgttgccttc 360

gagaaaggtg tcaagggcca gggtggcatg ccggcctcat tctgcaaaga agagcatggc 420

ccgcgcaagc tccgcgtggc tgtcttttgc ggtttggtct ttggcagttt ttccgaggac 480

gtgcccagca ttgaggatta ccttggccct gagatttgcg agcgcataga gcgcgtgctg 540

cacaagcccg tagaagtcat cggtcgcttc acgcaaaagc tgcctaacaa ctggaagctc 600

tacttcgaga acgtgaagga cagctatcac gccagcctcc tgcatatgtt cttcaccacc 660

ttcgagctga atcgcctctc acaaaaaggc ggtgtcatcg tcgacgagtc gggtggccac 720

catgtgagct attccatgat cgatcgcggc gccaaagacg actcgtacaa ggaccaggcc 780

atccgctccg acaacgagcg ttaccggctc aaagatccta gccttctaga gggctttgag 840

gagttcgagg acggcgtgac cctgcagatc ctttctgtgt tccctggctt tgtgctgcag 900

cagattcaga acagcatcgc cgtgcgtcag ttgctgccca agagcatctc cagctcggaa 960

ctcaactgga cctatcttgg ctatgcagat gacagtgccg agcaacgcaa ggtcagactc 1020

aaacaggcca accttatcgg cccggccgga ttcatttcca tggaagacgg agctgtcggt 1080

ggattcgtgc agcgtggcat cgcaggcgct gccaaccttg atgcagtcat cgagatgggc 1140

ggagaccacg aaggctctag cgagggccgc gccacggaaa cctcggtacg cggcttttgg 1200

aaggcctacc gcaagcatat gggacaggag atgcaagcat ga 1242

<210> 12
<211> 465
<212> DNA
<213> Comamonas sp.

<400> 12
atgatcaatg aaattcaaat cgcggccttc aatgccgcct acgcgaagac catagacagt 60

gatgcaatgg agcaatggcc aacctttttc accaaggatt gccactattg cgtcaccaat 120

gtcgacaacc atgatgaggg acttgctgcc ggcattgtct gggcggattc gcaggacatg 180

ctcaccgacc gaatttctgc gctgcgcgaa gccaatatct acgagcgcca ccgctatcgc 240

catatcctgg gtctgccttc gatccagtca ggcgatgcaa cacaggccag cgcttccact 300

ccgttcatgg tgctgcgcat catgcataca ggggaaacag aggtctttgc cagcggtgag 360

tacctcgaca aattcaccac gatcgatggc aagttacgtc tgcaagaacg catcgcggtt 420

tgcgacagca cggtgacgga cacgctgatg gcattgccgc tatga 465

<210> 13
<211> 948
<212> DNA
<213> Comamonas sp.

<400> 13
atgacaatag tgcaccgtag attggctttg gccatcggcg atccccacgg tattggccca 60

gaaatcgcac tgaaagctct ccagcagctg tctgtcaccg aaaggtctct tatcaaggtc 120

tatggacctt ggagcgctct cgagcaagcc gcacgggttt gcgaaatgga gccgcttctt 180

caagacatcg ttcacgagga agccggcaca cttacacaac cagttcaatg gggagaaatc 240

accccgcagg ctggtctatc tacggtgcaa tccgcaacag cggctatccg agcgtgcgaa 300

aacggcgaag tcgatgccgt cattgcctgc cctcaccatg aaacggccat tcaccgcgca 360

ggcatagcgt tcagcggcta cccatctttg ctcgccaatg ttcttggcat gaacgaagac 420

caggtattcc tgatgctggt gggggctggc ctgcgcatag tgcatgtcac tttgcatgaa 480

agcgtgcgca gcgcattgga gcggctctct cctcagttgg tggtcaacgc ggcgcaggct 540

gccgtgcaga catgcacctt actcggagtg cctaaaccaa aagtcgctgt attcgggatc 600

aaccctcatg catctgaagg acagttgttc ggcctggagg actcccagat caccgttccc 660

gctgtcgaga cactgcgcaa gcgcggccta gcagtagacg gccccatggg agctgacatg 720

gttctggcac agcgcaagca cgacctgtat gtagccatgc tgcacgatca gggccatatc 780

cccatcaagc tgctggcacc taacggagcc agcgcactat ctatcggtgg cagggtggtg 840

ctttccagcg tgggccatgg cagcgccatg gacattgccg gccgtggcgt ggctgacgcc 900

acggccctcc tacgcacaat agccctactc ggagcccaac cggtctga 948

<210> 14
<211> 1011
<212> DNA
<213> Comamonas sp.

<400> 14
atgaaccacc agatccatat ccacgactcc gatatcgcgt tcccctgcgc gcccgggcaa 60

tccgtactgg atgcagctct gcaggccggc atcgagctgc cctattcctg ccgcaaaggt 120

agctgtggca actgtgcgag tacgctgctc gacggaaata ttgcctcctt caatggcatg 180

gccgtgcgaa acgaactctg cgcctcggaa caagtgctgc tgtgcggctg cactgcagcc 240

agcgatatac gtatccaccc gagctccttt cgccgtctcg acccggaagc ccgaaaacgt 300

tttacggcca aggtgtacag caatacactg gcggcacccg atgtctcgct gctgcgcctg 360

cgcctgcctg tgggcaagcg cgccaaattt gaagccggcc aatacctgct gattcacctc 420

gacgacgggg aaagccgcag ctactctatg gccaatccac cccatgagag cgatggcatc 480

acattgcatg tcaggcatgt acctggtggt cgcttcagca ctatcgttca gcagttgaag 540

tctggtgaca cattggatat cgaactgcca ttcggcagca tcgcactgaa gcctgatgac 600

gcaaggcccc tgatttgcgt tgcgggtggc acgggatttg cgcccattaa atccgttctt 660

gatgacttag ccaaacgcaa ggttcagcgc gacatcacgc tgatctgggg ggctcgcaac 720

ccctcgggcc tgtatcttcc tagcgccatc gacaagtggc gcaaagtctg gccacagttt 780

cgctacattg cagccatcac cgacctaggc gatatgcctg cggatgctca cgcaggtcgg 840

gtggatgacg cgctacgcac tcactttggc aacctgcacg atcatgtggt gcactgctgt 900

ggctcaccag ctctggttca atcagtgcgc acagccgctt ccgatatggg cctgcttgca 960

caggacttcc acgcggatgt ttttgcgaca ggcccgactg gtcaccacta g 1011

<210> 15
<211> 720
<212> DNA
<213> Pseudomonas putida KT2440

<400> 15
atgcccgccc aggacaacag ccgcttcgtg atccgtgatc gcaactggca ccctaaagcc 60

cttacgcctg actacaagac ctccgttgcc cgctcgccgc gccaggcact ggtcagcatt 120

ccgcagtcga tcagcgaaac cactggtccg gacttttccc atctgggctt cggcgcccac 180

gaccatgacc tgctgctgaa cttcaataac ggtggcctgc ccattggcga gcgcatcatc 240

gtcgccggcc gtgtcgtcga ccagtacggc aagcctgtgc cgaacacttt ggtggagatg 300

tggcaagcca acgccggcgg ccgctatcgc cacaagaacg atcgctacct ggcgcccctg 360

gacccgaact tcggtggtgt tgggcggtgt ctgaccgacc gtgacggcta ttacagcttc 420

cgcaccatca agccgggccc gtacccatgg cgcaacggcc cgaacgactg gcgcccggcg 480

catatccact tcgccatcag cggcccatcg atcgccacca agctgatcac ccagttgtac 540

ttcgaaggtg acccgctgat cccgatgtgc ccgatcgtca agtcgatcgc caacccgcaa 600

gccgtgcagc agttgatcgc caagctcgac atgagcaacg ccaacccgat ggactgcctg 660

gcctaccgct ttgacatcgt gctgcgcggc cagcgcaaga cccacttcga aaactgctga 720

<210> 16
<211> 606
<212> DNA
<213> Pseudomonas putida KT2440

<400> 16
atgccaatcg aactgctgcc ggaaacccct tcgcagactg ccggccccta cgtgcacatc 60

ggcctggccc tggaagccgc cggcaacccg acccgcgacc aggaaatctg gaactgcctg 120

gccaagccag acgccccggg cgagcacatt ctgctgatcg gccacgtata tgacggaaac 180

ggccacctgg tgcgcgactc gttcctggaa gtgtggcagg ccgacgccaa cggtgagtac 240

caggatgcct acaacctgga aaacgccttc aacagctttg gccgcacggc taccaccttc 300

gatgccggtg agtggacgct gcaaacggtc aagccgggtg tggtgaacaa cgctgctggc 360

gtgccgatgg cgccgcacat caacatcagc ctgtttgccc gtggcatcaa catccacctg 420

cacacgcgcc tgtatttcga tgatgaggcc caggccaatg ccaagtgccc ggtgctcaac 480

ctgatcgagc agccgcagcg gcgtgaaacc ttgattgcca agcgttgcga agtggatggg 540

aagacggcgt accgctttga tatccgcatt cagggggaag gggagaccgt cttcttcgac 600

ttctga 606

## Description

### Technical Field

The present invention relates to a protocatechuate 4,5-ring-cleaving enzyme gene-disrupted strain, to recombinant plasmids comprising a gene coding for an enzyme participating in a multistage reaction process for fermentative production of 3-carboxy-cis,cis-muconic acid and/or 3-carboxymuconolactone from terephthalic acid via protocatechuic acid, to transformants incorporating the recombinant plasmid in the disrupted strain, and to a process for industrial production of 3-carboxy-cis,cis-muconic acid and/or 3-carboxymuconolactone using the same.

### Background Art

Terephthalic acid is an aromatic compound separated from petroleum components, and it is cheaply mass-produced as a starting material for PET. Development of new biodegradable functional plastics using terephthalic acid as the starting material will allow copolymerization with petroleum-based polymer materials such as PET, to permit the development of polymer materials with excellent biodegradability.

The present inventors have found that the terephthalic acid-degrading microorganism, Comamonas sp. E6, can completely degrade terephthalic acid via 2H-pyran-2-one-4,6-dicarboxylic acid after first converting it to protocatechuic acid (Patent document 1). There have also been reported a recombinant vector comprising the genes coding for terephthalate dioxygenase (TPA-DOX), 1,2-dihydroxy-3,5-cyclohexadiene-1,4-dicarboxylate dehydrogenase (DCD-dehydrogenase), terephthalate transporter (TPA transporter) and a positive regulator, by removing the genes coding for protocatechuate 4,5-dioxygenase and 4-carboxy-2-hydroxy-6-semialdehyde muconate dehydrogenase from the chromosomal DNA of the microorganism, transformants containing the vector, and a method of producing 2H-pyran-2-one-4,6-dicarboxylic acid from terephthalic acid using the transformants (Japanese Patent Application No. 2005-298242).

The present inventors have also reported a process for fermentative production of 3-carboxy-cis,cis-muconic acid and/or 3-carboxymuconolactone from plant components such as vanillin, vanillic acid and protocatechuic acid, via a multistage enzyme reaction (Japanese Patent Application No. 2006-218524).

In the process, terephthalic acid is converted to 3-carboxy-cis and cis-muconic acid via 1,2-dihydroxy-3,5-cyclohexadiene-1,4-dicarboxylate and further via protocatechuic acid, and the 2,3-bond, 3,4-bond or 4,5-bond of protocatechuic acid is cleaved, depending on the type of microorganism (hereunder, 2,3-bond cleavage, 3,4-bond cleavage and 4,5-bond cleavage of protocatechuic acid will be referred to as "2,3-ring cleavage", "3,4-ring cleavage" and "4,5-ring cleavage", respectively).
In the presence of certain microorganisms, the 3-carboxy-cis,cis-muconic acid as the 3,4-ring cleavage product of protocatechuic acid is further catabolized via 3-carboxymuconolactone or 4-carboxymuconolactone.

No process has been known to date for fermentative production of 3-carboxy-cis,cis-muconic acid using terephthalic acid as the starting material.

### Disclosure of the Invention

It is an object of the present invention to provide a process for industrial-scale fermentative production of 3-carboxy-cis,cis-muconic acid from terephthalic acid via protocatechuic acid, and/or a process for fermentative production of 3-carboxy-cis,cis-muconic acid from terephthalic acid via protocatechuic acid and acid treatment thereof to obtain 3-carboxymuconolactone on an industrial scale.

In order to obtain 3-carboxy-cis,cis-muconic acid efficiently, it is necessary to disrupt genes having 2,3-ring cleavage function, 3,4-ring cleavage function or 4,5-ring cleavage function, or to disrupt genes that further metabolize 3-carboxy-cis,cis-muconic acid.

The present inventors have conducted ardent research on this subject, and as a result have considered that disrupting the cleavage activity of protocatechuate 4,5-ring-cleaving enzyme would completely disrupt the conversion process from protocatechuic acid to 2H-pyran-2-one-4,6-dicarboxylic acid, and have therefore generated protocatechuate 4,5-ring-cleaving enzyme gene-disrupted strains in which the cleavage activity of protocatechuate 4,5-ring-cleaving enzyme has been disrupted. It was further found that it is possible, using the disrupted strains, to produce 3-carboxy-cis,cis-muconic acid and/or its acid treatment product, 3-carboxymuconolactone, from terephthalic acid at high yield and inexpensively.

Specifically, (1) the present invention provides a protocatechuate 4,5-ring-cleaving enzyme gene-disrupted strain in which the gene coding for:
(a) the amino acid sequence set forth in SEQ ID NO: 1 or 3, or
(b) the amino acid sequence set forth in SEQ ID NO: 1 or 3 which has a deletion, substitution, addition and/or insertion of one or more amino acids and exhibits protocatechuate 4,5-ring cleavage activity, present in the chromosomal DNA of microbial cells, has been disrupted.
(2) The present invention further provides a protocatechuate 4,5-ring-cleaving enzyme gene-disrupted strain in which a gene that comprises:
   (a) the nucleotide sequence set forth in SEQ ID NO: 2 or 4; or
   (b) a nucleotide sequence hybridizing with DNA consisting of a nucleotide sequence complementary to the nucleotide sequence of (a), under stringent conditions, and coding for an enzyme with protocatechuate 4,5-ring cleavage activity, present in the chromosomal DNA of microbial cells, has been disrupted.
(3) The invention further provides a gene-disrupted strain according to (1) or (2), in which the protocatechuate 4,5-ring-cleaving enzyme gene has been disrupted by homologous recombination between a gene coding for:
   (a) the amino acid sequence set forth in SEQ ID NO: 1 or 3, or
   (b) the amino acid sequence set forth in SEQ ID NO: 1 or 3 which has a deletion, substitution, addition and/or insertion of one or more amino acids and exhibits protocatechuate 4,5-ring cleavage activity, present in the chromosomal DNA of microbial cells, and homologous recombination DNA having a DNA sequence that can undergo homologous recombination with the gene and lacking protocatechuate 4,5-ring cleavage activity.
(4) The invention further provides a gene-disrupted strain according to any one of (1)-(3), wherein the parent strain of the protocatechuate 4,5-ring-cleaving enzyme gene-disrupted strain is a Comamonas sp. bacterium.
(5) The invention further provides a gene-disrupted strain according to (4), wherein the Comamonas bacterium is Comamonas sp. E6.
(6) The invention further provides a recombinant plasmid comprising a terephthalate dioxygenase gene (TPA-DOX gene), NADPH-reductase gene, 1,2-dihydroxy-3,5-cyclohexadiene-1,4-dicarboxylate dehydrogenase gene (DCD dehydrogenase gene), positive regulator gene, terephthalate transporter gene (TPA transporter gene) and protocatechuate 3,4-dioxygenase gene (pcaHG gene).
(7) The invention further provides a transformant obtained by introducing a recombinant plasmid according to (6) into a gene-disrupted strain according to any one of (1)-(5).
(8) The invention further provides a process for production of 3-carboxy-cis,cis-muconic acid and/or 3-carboxymuconolactone, characterized by culturing a transformant according to (7) in the presence of terephthalic acid.

According to the invention it is possible to accomplish high-yield and inexpensive fermentative production of 3-carboxy-cis,cis-muconic acid and/or 3-carboxymuconolactone from terephthalic acid.

### Brief Description of the Drawings

Fig. 1 is a 5.1-kbSalI-XhoI restriction enzyme and ORF map.
Fig. 2 shows the positional relationship between the pCS18 10-kb SacI fragment and the pPV10 10-kb SalI fragment.
Fig. 3 shows the protocatechuate 4,5-cleavage enzyme gene group from Comamonas sp. E6.
Fig. 4 shows the pmdB1 disrupted strain plasmid pDBKM.
Fig. 5 shows disruption of the pmdB1 gene in Comamonas sp. E6. 5(A) is an illustration of a method of constructing an E6 pmdB1 gene-disrupted strain, and 5(B) shows the results of Southern hybridization analysis of the pmdB1 gene-disrupted strain.
Fig. 6 is an illustration showing construction of the recombinant plasmid pKHG.
Fig. 7 is an illustration showing construction of the recombinant plasmid pKHG/C.
Fig. 8 is an illustration showing construction of the recombinant plasmid pKTphHG/C.

### Best Mode for Carrying Out the Invention

The gene-disrupted strain of the invention may be one in which at least one function of the 2,3-ring cleavage function, 3,4-ring cleavage function and 4,5-ring cleavage function is disrupted. Alternatively, the gene-disrupted strain of the invention may retain the 3,4-ring cleavage function, in which case the function of further metabolizing 3-carboxy-cis,cis-muconic acid must be disrupted. In order to disrupt the activity of metabolizing carboxy-cis,cis-muconic acid, the 3-carboxymuconolactonizing enzyme or 4-carboxymuconolactonizing enzyme may be disrupted.

Production of 3-carboxy-cis,cis-muconic acid according to the invention is accomplished by introducing the gene-disrupted strain of the invention into, for example, i) a host microorganism having a function of metabolizing terephthalic acid to protocatechuic acid (terephthalate assimilation), and lacking a terephthalate 2,3-ring cleavage function but having a terephthalic acid 3,4-ring cleavage function or 4,5-ring cleavage function, or ii) a host microorganism lacking terephthalate assimilation but having "protocatechuate assimilation" by a protocatechuate 2,3-ring cleavage function, 3,4-ring cleavage function or 4,5-ring cleavage function.

The invention will now be explained in detail using a gene-disrupted strain with disrupted protocatechuate 4,5-ring cleavage function as an example.

### (I) Obtaining gene coding for protocatechuate 4,5-ring-cleaving enzyme (protocatechuate 4,5-dioxygenase)

For the purpose of the invention, the protocatechuate 4,5-ring cleavage enzyme gene group will be referred to as "pmd gene group." Of the pmd gene group, the gene coding for the α-subunit of the enzyme having dioxygenase activity that cleaves the protocatechuate 4,5-ring for conversion to 4-carboxy-2-hydroxy-6-semialdehyde muconate will be referred to as "pmdA1" (the amino acid sequence set forth in SEQ ID NO: 3 and the nucleotide sequence set forth in SEQ ID NO: 4), and the gene coding for the β-subunit of the same will be referred to as "pmdB1" (the amino acid sequence set forth in SEQ ID NO: 1 and the nucleotide sequence set forth in SEQ ID NO: 2).

Also, the gene coding for the enzyme with dehydrogenase activity that cleaves 4-carboxy-2-hydroxy-6-semialdehyde muconate for conversion to 2H-pyran-2-one-4,6-dicarboxylic acid will be referred to as "pmdC".

The method of obtaining the gene coding for protocatechuate 4,5-ring-cleaving enzyme is not particularly restricted, and for example, the gene may be obtained by preparing a suitable probe or primer based on the data for the nucleotide sequence of the gene, and screening a cDNA library or genomic DNA library for the strain, using the probe or primer.

The gene for protocatechuate 4,5-ring-cleaving enzyme may also be obtained by PCR. The chromosomal DNA or cDNA library of the strain may be used as template for PCR with a pair of primers designed so as to amplify the nucleotide sequence of the gene. The PCR reaction conditions may be set as appropriate, and for example, it may be carried out under conditions in which reaction for 30 seconds at 94°C (denaturation), 30 seconds to 1 minute at 55°C (annealing) and 2 minutes at 72°C (extension) as one cycle, is carried out for 30 cycles followed by reaction for 7 minutes at 72°C. The amplified DNA fragment may then be cloned in a suitable vector. The vector is preferably selected as a vector that is autoreplicating in E. coli and that is incapable of extrachromosomal autoreplication in Comamonas sp.

The procedures for preparation of the probe or primer, construction of the cDNA library, screening of the cDNA library and cloning of the target gene may be known to those skilled in the art, and for example, they may be carried out according to the methods described in Molecular Cloning: A laboratory Manual, 2nd Edition, Cold Spring Harbor Laboratory, Cold Spring Harbor, NY., 1989 and Current Protocols in Molecular Biology, Supplement pp.1-38, John Wiley & Sons (1987-1997).

### (II) Construction of gene-disrupted strain

The gene-disrupted strain of the invention is a disrupted strain wherein protocatechuate 4,5-ring cleavage has been selectively disrupted. More specifically, the gene coding for: (a) the amino acid sequence set forth in SEQ ID NO: 1 or 3, or (b) the amino acid sequence set forth in SEQ ID NO: 1 or 3 which has a deletion, substitution, addition and/or insertion of one or more amino acids and exhibits protocatechuate 4,5-ring cleavage activity, present in the chromosomal DNA of microbial cells, has been disrupted.

The gene may be a gene that comprises (a) the nucleotide sequence set forth in SEQ ID NO: 2 or 4; or (b) a nucleotide sequence hybridizing with DNA consisting of a nucleotide sequence complementary to the nucleotide sequence of (a) under stringent conditions, and coding for an enzyme with protocatechuate 4,5-ring cleavage activity.

Specifically, the gene to be disrupted may be the pmdB1 gene set forth in SEQ ID NO: 2, the pmdA1 gene set forth in s SEQ ID NO: 4, or both of these genes.

There are no particular restrictions on the range of "one or more" in the phrase "amino acid sequence having a deletion, substitution, addition and/or insertion of one or more amino acids" used throughout the present specification, and it may be, for example, 1-20, preferably 1-10, more preferably 1-7 and most preferably about 1-3.

The phrase "stringent hybridization conditions" used in the present specification means "highly stringent conditions" under which a DNA chain can hybridize to another DNA chain which is highly complementary to the DNA chain, and which is designed so as to exclude significantly mismatched DNA hybridization, or "moderately stringent conditions" under which DNA double strands can form with a greater degree of base pair mismatching than is possible under "highly stringent conditions". As specific examples of "highly stringent conditions" there may be mentioned 0.015 M sodium chloride and 0.0015 M sodium citrate at 65-68°C, or 0.015 M sodium chloride, 0.0015 M sodium citrate, and 50% formamide at 42°C. As specific examples of "moderately stringent conditions" there may be mentioned 0.015 M sodium chloride and 0.0015 M sodium citrate at 50-65°C, or 0.015 M sodium chloride, 0.0015 M sodium citrate, and 20% formamide at 37-50°C.

As specific hybridizing DNA there may be mentioned DNA having at least 60%, preferably at least 70%, more preferably at least 80%, even more preferably at least 90%, yet more preferably at least 95% and most preferably at least 97% homology with a polynucleotide containing the nucleotide sequence set forth in SEQ ID NO: 2 or 4, as calculated using analysis software such as BLAST [J. Mol. Biol., 215, 403 (1990)] or FASTA [Methods in Enzymology, 183, 63 (1990)].

Throughout the present specification, "having protocatechuate 4,5-ring cleavage activity" means activity equivalent to an enzyme that cleaves the protocatechuate 4,5-ring to convert protocatechuic acid to 4-carboxy-2-hydroxy-6-semialdehyde muconate.

The gene-disrupted strain of the invention can be generated by introducing a mutation and/or a selective marker into the gene coding for protocatechuate 4,5-ring-cleaving enzyme obtained by PCR or cloning, to obtain DNA lacking protocatechuate 4,5-ring cleavage activity, and then using the DNA for homologous recombination.

The method for introducing a selective marker into the gene coding for protocatechuate 4,5-ring-cleaving enzyme may be, for example, a method in which the gene is cut with an appropriate restriction enzyme and then an unrelated gene, preferably a selective marker gene that allows selection of strains that have undergone homologous recombination, is inserted. When no suitable restriction enzyme site is present, a suitable restriction enzyme site may be introduced by PCR or the like. As selective markers there are preferred drug resistance markers, and as examples there may be mentioned kanamycin resistance genes, ampicillin resistance genes and tetracycline resistance genes.

The method for introducing mutations into the gene coding for protocatechuate 4,5-ring-cleaving enzyme may be any of the numerous known methods, such as recombinant DNA techniques for manipulating DNA nucleotide sequences (Sambruck, J., Fritsch, E. F. and Maniatis, T. (1989) Molecular Cloning: A Laboratory Manual, Cold Spring Harbor Laboratory, Cold Spring Harbor, NY), and PCR-applied techniques (Ling M.M. and Robinson BH., Anal. Biochem. 254(2): 157-78, 1997).

The DNA lacking protocatechuate 4,5-ring cleavage activity, used to generate the gene-disrupted strain of the invention, may be any that undergoes homologous recombination with a gene coding for the corresponding protocatechuate 4,5-ring-cleaving enzyme on the chromosome under physiological conditions, i.e. in microbial cells, having sufficient homology to thus allow disruption of the protocatechuate 4,5-ring-cleaving enzyme gene. The homology is preferably 80% or greater, more preferably 90% or greater and most preferably 95% or greater. The DNA used for homologous recombination may also be a portion of the protocatechuate 4,5-ring-cleaving enzyme gene, so long as it undergoes homologous recombination with a gene coding for the corresponding protocatechuate 4,5-ring-cleaving enzyme on the chromosome under physiological conditions, i.e. in microbial cells, thereby allowing disruption of the protocatechuate 4,5-ring-cleaving enzyme gene. The portion referred to here may be a length of preferably 50 or more nucleotides, and more preferably 100 or more nucleotides.

In order to generate a gene-disrupted strain by homologous recombination, first there is constructed DNA containing the nucleotide sequence of the protocatechuate 4,5-ring-cleaving enzyme gene or recombinant DNA comprising the mutated DNA having an appropriate selective marker inserted therein. When a drug resistance marker is used as the selective marker, it is necessary to select a resistance gene for a drug to which the wild type strain prior to homologous recombination is sensitive. This will allow discernment between strains that have undergone homologous recombination and strains that have not, based on growth in the presence of an antibiotic. Next, the DNA having the selective marker inserted into the gene sequence is introduced into the strain by electroporation or the like, and then selection is carried out using the marker to incorporate the target gene into the host microorganism chromosomes by homologous recombination.

The parent strain of the protocatechuate 4,5-ring-cleaving enzyme gene-disrupted strain is not particularly restricted so long as it is a soil bacterium of Comamonas, Pseudomonas, Bacillus, Lactobacillus, Streptococcus, Saccharomyces, Candida or the like. The parent strain may be a strain derived from a microorganism that is capable of "terephthalate assimilation", or a strain derived from a microorganism that is incapable of "terephthalate assimilation" but is capable of "protocatechuate assimilation".

A microorganism that is capable of terephthalate assimilation is able to metabolize terephthalic acid to protocatechuic acid. As examples of strains capable of terephthalate assimilation, there may be mentioned Comamonas sp. E6, Pseudomonas putida PPY1100, Comamonas testosteroni (C. testosteroni) T-2, C. testosteroni YZW-D, Derftia tsuruhatensis T7, Rhodococcus sp. DK17 and Rhodococcus jostii RHA1. Of these, Comamonas sp. E6 is a strain that retains ability to metabolize terephthalic acid to protocatechuic acid and ability to metabolize protocatechuic acid to 2H-pyran-2-one-4,6-dicarboxylic acid (4,5-ring cleavage function), but does not retain 2,3-ring cleavage function and 3,4-ring cleavage function. When Comamonas sp. E6 was used as the parent strain, the production efficiency for 3-carboxy-cis,cis-muconic acid was roughly equivalent to a fermentative production system from vanillic acid to 2H-pyran-2-one-4,6-dicarboxylic acid (Japanese Unexamined Patent Publication No. 2005-278549) or a fermentative production system to 3-carboxy-cis,cis-muconic acid and/or 3-carboxymuconolactone (Japanese Patent Application No. 2006-218524).

A microorganism that is not capable of terephthalate assimilation but is capable of protocatechuate assimilation has a 2,3-ring cleavage function, 3,4-ring cleavage function or 4,5-ring cleavage function. As examples of such microorganisms there may be mentioned microorganisms belonging to the genus Pseudomonas, Bacillus, Burkholderia and Agrobacterium.

A gene-disrupted strain with disrupted protocatechuate 2,3-ring cleavage function or 3,4-ring cleavage function can also be generated in the same manner as the aforementioned 4,5-ring cleavage function-disrupted strain.

A microorganism capable of terephthalate assimilation will generally have more developed terephthalate uptake and metabolism compared to a microorganism incapable of terephthalate assimilation, and higher conversion efficiency would be expected.

Incidentally, 3-carboxy-cis,cis-muconic acid can also be efficiently produced using a strain that is not a gene-deleted strain according to the invention but which retains protocatechuate 3,4-ring cleavage function and does not further catabolize the 3-carboxy-cis,cis-muconic acid cleavage product. Pseudomonas putida PPY1100 may be mentioned as an example of such a strain, but the reaction efficiency is slightly lower with Pseudomonas putida PPY1100 compared to Comamonas sp. E6.

For even more efficient production of 3-carboxy-cis,cis-muconic acid, it is more preferred to use a gene-disrupted strain having disrupted protocatechuate 2,3-and 4,5-ring cleavage activity, and also having disrupted 3-carboxy-cis,cis-muconic acid metabolism activity in order to completely eliminate the possibility of further catabolism of 3-carboxy-cis,cis-muconic acid.

### (III) Preparation of transformants incorporating 3-carboxy-cis,cis-muconic acid fermentative production plasmid

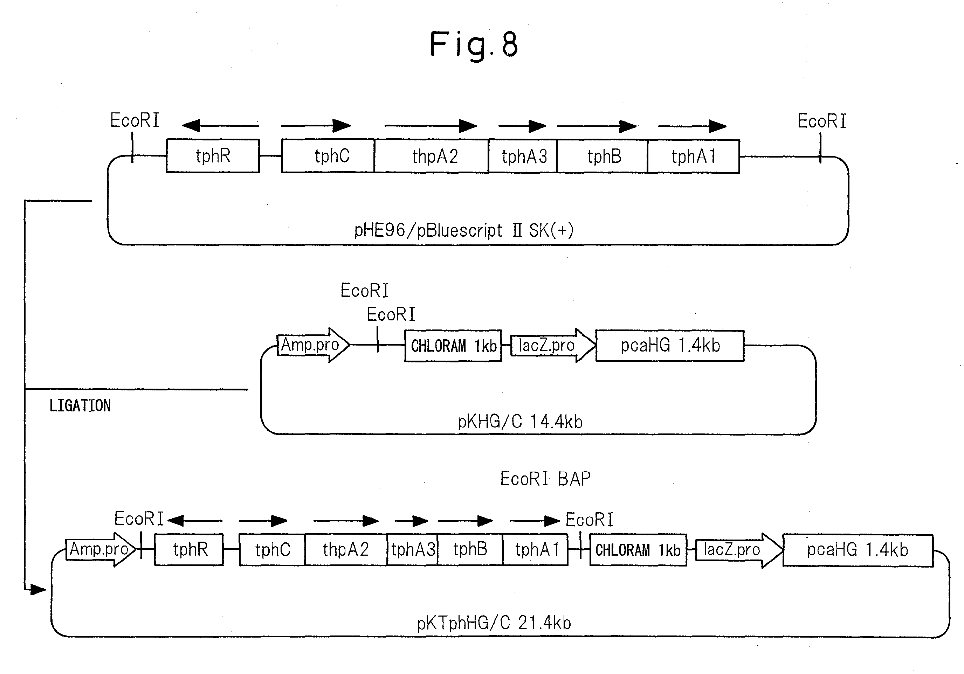

The plasmid is a plasmid comprising genes for enzymes that catalyze a multistage process for production of 3-carboxy-cis,cis-muconic acid from terephthalic acid via protocatechuic acid (positive regulator, TPA-transporter, TPA-DOX, DCD-dehydrogenase, NADPH-reductase, protocatechuate 3,4-ring cleavage gene), in that order from the upstream end (Fig. 8).

The positive regulator gene (tphR) is the DNA molecule set forth in SEQ ID NO: 11 in Japanese Patent Application No. 2005-298242 (SEQ ID NO: 9 in the present specification), the TPA-transporter gene (tphC) is the DNA molecule set forth in SEQ ID NO: 13 in the same specification (SEQ ID NO: 10 in the present specification), the TPA-DOX genes (tphA2, tphA3) are the DNA molecules set forth in SEQ ID NO: 2 and 4 in the same specification (SEQ ID NO: 11 and 12, respectively, in the present specification), the DCD-dehydrogenase gene (tphB) is the DNA molecule set forth in SEQ ID NO: 6 in the same specification (SEQ ID NO: 13 in the present specification), and the NADPH-reductase gene (tphA1) is the DNA molecule set forth in SEQ ID NO: 8 in the same specification (SEQ ID NO: 14 in the present specification). The protocatechuate 3,4-ring cleavage genes (pcaH and G) used for the invention are DNA fragments obtained from Pseudomonas putida KT2440, and the nucleotide sequence of the PcaH gene is set forth in SEQ ID NO: 1 of Japanese Patent Application No. 2006-218524 while the nucleotide sequence of the PcaG gene is set forth in SEQ ID NO: 3 of the same specification (SEQ ID NO: 15 and 16 of the present specification). In the present specification, tphR, tphC, tphA2, tphA3, tphB and tphA1 will also be collectively referred to as "Tph gene cluster" or "Tph gene group".

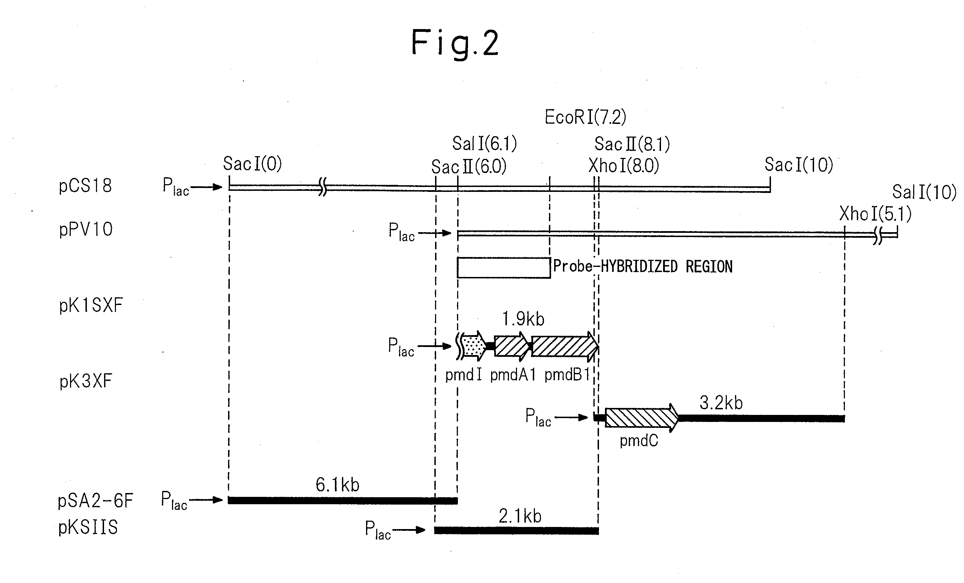

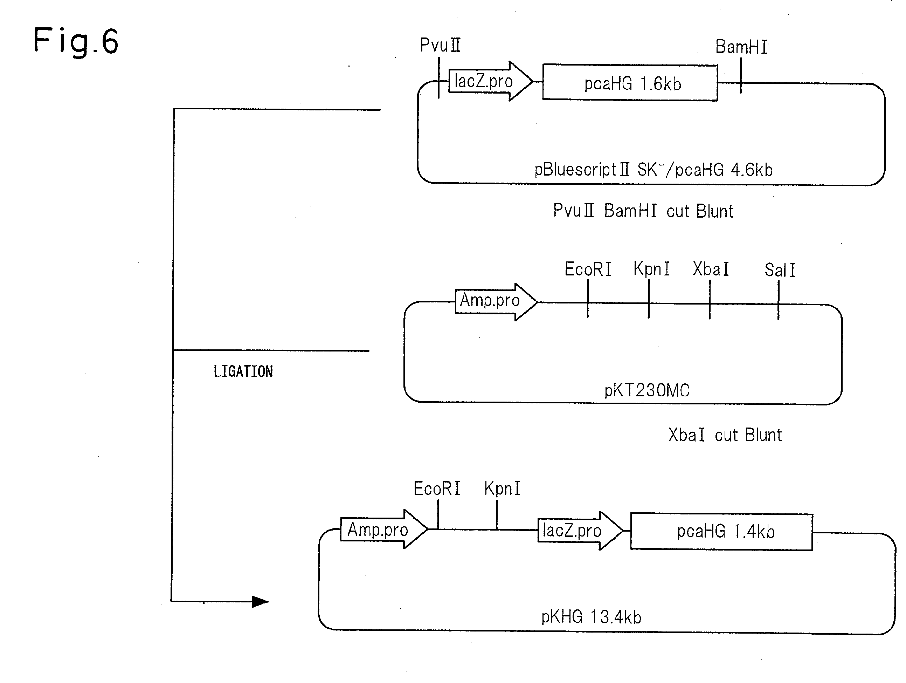

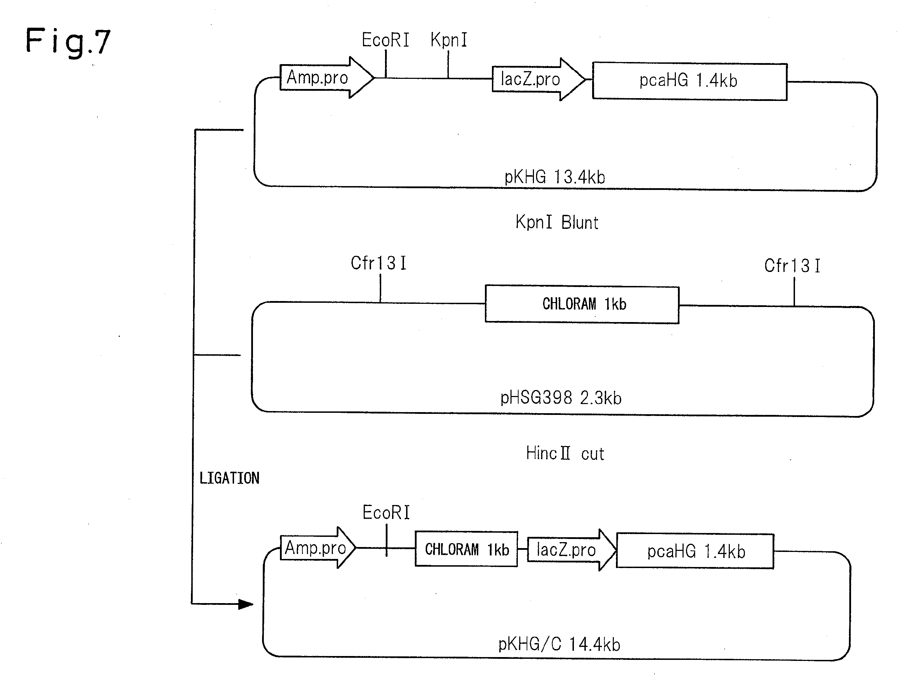

Specifically, the plasmid for fermentative production of 3-carboxy-cis,cis-muconic acid from terephthalic acid according to the invention may be constructed as illustrated in Figs. 6 to 8.
(1) First, the PcaH gene (SEQ ID NO: 1) and PcaG gene (SEQ ID NO: 2) listed in Japanese Patent Application No. 2006-218524 are ligated to a multicloning site in the gene coding for the α-fragment of LacZ, present downstream from the pBluescript LacZ promoter, using a known ligase, to construct recombinant plasmid pBluescript II SK⁻/pcaHG.
   Next, a known ligase is used to ligate a DNA fragment obtained by end treatment after cutting pBluescript II SK⁻/pcaHG with restriction enzymes PvuII and BamHI, with a DNA fragment obtained by end treatment after cutting an Amp promoter-containing plasmid with restriction enzyme XbaI, to construct recombinant plasmid pKHG (Fig. 6).
(2) Next, a known ligase is used to ligate a DNA fragment obtained by end treatment after cutting a chloramphenicol resistance gene-containing plasmid with restriction enzyme Cfr13I, with a DNA fragment obtained by end treatment after cutting pKHG with restriction enzyme KpnI, to construct recombinant plasmid pKHG/C (Fig. 7).
(3) Also, a known ligase may be used to ligate a DNA fragment obtained by cutting recombinant plasmid pHE96/pBluescript II SK (+), having tphR, tphC, tphA2, tphA3, tphB and tphA1 ligated in that order from the upstream end and shown in Fig. 2 of Japanese Patent Application No. 2005-298242, with restriction enzyme EcoRI, with a DNA fragment obtained by end treatment after cutting the pKHG/C obtained in (2) with EcoRI, to construct recombinant plasmid pKTphHG/C.

A known method such as a protoplast method, competent cell method or electroporation method may be used for transformation of the gene-disrupted strain of (I) using the recombinant plasmid pKTphHG/C.

Selection of transformants may be accomplished based on a selective marker for the plasmid used, such as drug resistance acquired by DNA recombination in the transformants. The transformants containing the recombinant plasmid of interest are preferably selected from among the transformants by colony hybridization using a partial DNA fragment of the gene as the probe. Labeling of the probe may be carried out using a radioactive isotope, digoxigenin, an enzyme or the like.

### (IV) the fermentative production of 3-carboxy-cis,cis-muconic acid and/or 3-carboxymuconolactone

The transformants of the invention of (II) may be cultured under appropriate conditions in the presence of terephthalic acid, using medium containing a carbon source, nitrogen source, metal salts, minerals, vitamins and the like. The pH of the medium may be a pH in a range that allows growth of the transformants, and the pH is preferably adjusted to about 6-8. The culturing conditions may be shake culturing or submerged culturing for 2-7 days at 15-40°C and preferably 28-37°C.

Ordinary isolation and purification methods for organic compounds may be used for isolation and purification of 3-carboxy-cis,cis-muconic acid from the cultured gene-disrupted strain. For example, upon completion of culturing, the cells are collected by centrifugal separation and suspended in aqueous buffer, and then disrupted using an ultrasonic disruptor or the like to obtain a cell-free extract. The target substance may be obtained by ordinary isolation and purification methods for organic compounds, from the supernatant obtained by centrifugal separation of the cell-free extract.

Acid treatment of the 3-carboxy-cis,cis-3-muconic acid obtained in this manner, or the culture solution containing the unpurified 3-carboxy-cis,cis-3-muconic acid, will allow conversion to 3-carboxymuconolactone at a high yield. The acid used is preferably hydrochloric acid at about pH 1-2.

The 3-carboxy-cis,cis-muconic acid and/or 3-carboxymuconolactone obtained by the production process of the invention, as a plastic material, chemical product material or the like, can exhibit functions different from 2H-pyran-2-one-4,6-dicarboxylic acid or higher functions thereof, and therefore can serve as a useful plastic material.

### Examples

The present invention will now be described in greater detail by examples, with the understanding that the invention is not limited to these examples.

### Example 1: Cloning of pmd gene group

### (1) Amplification of protocatechuate 4,5-dioxygenase gene by PCR and sequencing

A region of high homology was identified from alignment of the amino acid sequences deduced from the ligA gene of Sphingomonas paucimobilis SYK-6, the pcmA gene of Arthrobacter keyseri 12B, the pmdA1B1 gene of Comamonas testosteroni BR6020 and the fldVU gene of Sphingomonas sp. LB126, and PCR was conducted using the following αF/βR primer, with the total DNA of the Comamonas sp. E6 protocatechuate 4,5-dioxygenase gene as template.
αF of α-subunit (26 mer: ATGWSSCTGATGAARSCSGARAACCG; SEQ ID NO: 5)
βF of β-subunit (27 mer: GTSWTCCTGGTSTAYAACGAYCAYGCY; SEQ ID NO: 6); and
βR of β-subunit (25 mer: CCCTGMARCTGRTGGCTCATGCCGC; SEQ ID NO: 7).

As a result, amplification was seen at the predicted size of 900 bp. The PCR product was then used as template for PCR between βF-βR, using the nested primer βF. As a result, amplification was seen of a 450 bp fragment of the predicted size. The obtained PCR product was subcloned in pT7Blue vector to determine the nucleotide sequence of the 449 bp fragment. A homologous sequence search was conducted in the DDBJ database, and the gene coding for the Comamonas testosteroni BR6020 protocatechuate 4,5-dioxygenase β-subunit (DDBJ Accession No.: AF305325) showed 99% homology on the amino acid level. Also, homology of 68% was found with Sphingomonas paucimobilis SYK-6 ligB (DDBJ Accession No.: AB035122), and 66% with both Arthrobacter keyseri 12B pcmA (DDBJ Accession No.: AF331043) and Sphingomonas sp. LB126 fldU (DDBJ Accession No.: AJ277295).

### (2) Isolation of cosmid containing protocatechuate 4,5-dioxygenase gene

Positive clones were obtained from a cosmid library containing E6 SalI partial digestion fragments, by colony hybridization using the PCR product as the probe. The cosmid extracted from the obtained positive clones was digested with SalI and subjected to Southern hybridization with the same probe, which produced hybridization of 10 kb, and the cosmid was named pPV10.

### (3) Nucleotide sequences of pPV10-derived 1.9-kb SalI-XhoI fragment and 3.2-kb XhoI fragment

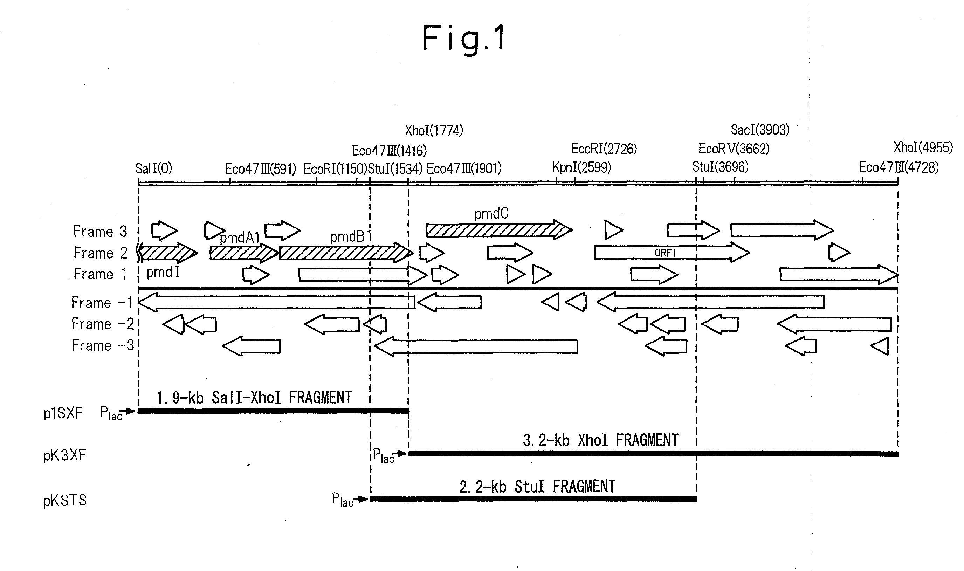

Clone pKS10F(R) was constructed by reciprocal bidirectional insertion of the pPV10 10-kb SalI fragment downstream from the pBluescript II KS (+) lac promoter. Three fragments of 1.9 kb, 3.2 kb and 4.9 kb, obtained by digestion of pKS10F with SalI and XhoI, were blunted and inserted into the SmaI site of pBluescript II KS(+). After reciprocal bidirectional insertion of these fragments downstream from the lac promoter, the obtained plasmids were named pK1SXF(R), pK3XF(R) and pK4XSF(R), respectively (Fig. 1). The full nucleotide sequences of the pK1SXF(R) 1.9-kb SalI-XhoI fragment and the 3.2-kb XhoI fragment in pK3X were determined. Sequencing of the pKSTS 2.2-kb StuI fragment also confirmed that these fragments are adjacent across XhoI (Fig. 1). In Fig. 1, Plac represents the lac promoter, with the arrow indicating the direction of transcription.

### (4) Isolation of region upstream from 10-kb SalI fragment

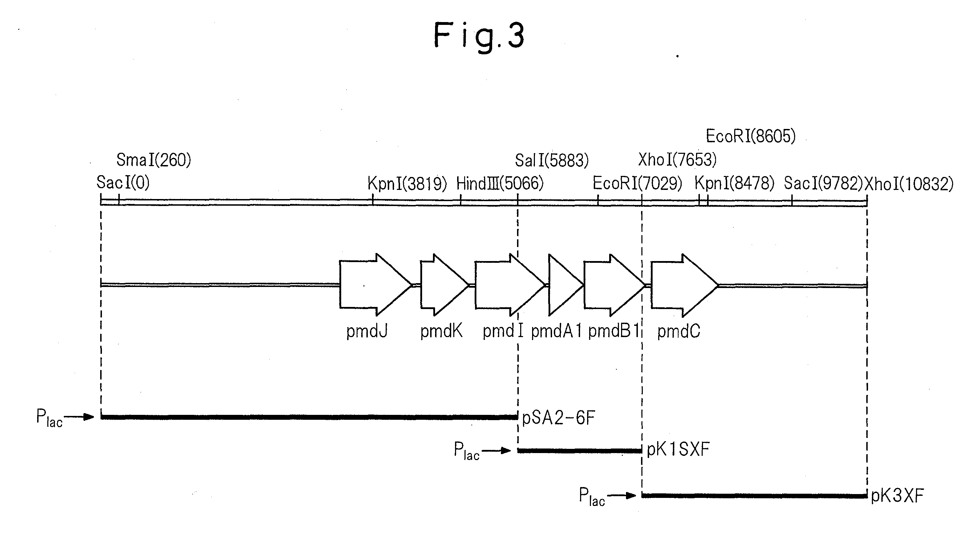

In order to isolate the region upstream from the pPV10 10-kb SalI fragment, a library was prepared by cloning of a SacI digest of E6 total DNA in charomid 9-36. A 1,150 bp fragment obtained by digestion of the 1.9-kb SalI-XhoI fragment with SalI and EcoRI was used for colony hybridization, to isolate pCS18 having a 10-kb SacI fragment containing pmdA1B1 (Fig. 2). After nucleotide sequencing to confirm that the pSC18 2.1-kb SacII fragment containing pmdA1B1 is adjacent to the 6.1-kb SacI-SalI fragment, clone pSA2-6F(R) obtained by blunting the 6.1-kb SacI-SalI fragment and reciprocal bidirectional insertion at the EcoRV site downstream from the pBluescript II KS(+) lac promoter was generated, and the full nucleotide sequence was determined (Fig. 3). SEQ ID NO: 8 includes the gene sequence comprising the genes pmdJ (base numbers 1-1029), pmdK (base numbers 1162-1845), pmdI (base numbers 1942-2934), pmdA1 (SEQ ID NO: 2), pmdB1 (SEQ ID NO: 4 and pmdC (base numbers 4427-5386), shown in Fig. 3.

### Example 2: Construction of pmdB1 gene-disrupted strain

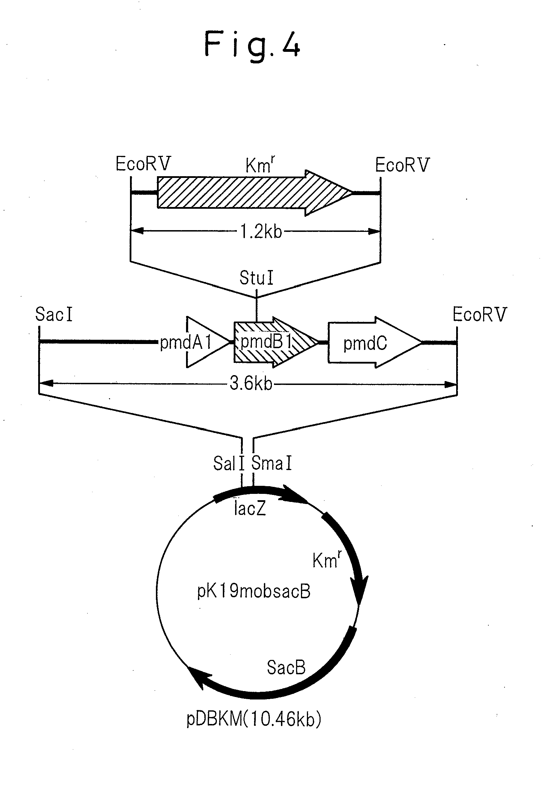

A 3.6-kb SalI-EcoRV fragment containing the pmdA1B1C gene was cut out from pKS10F and inserted into a SalI-EcoRV digest of pBluescript II KS(+) to obtain pSDB36.
It was then digested with StuI within the pmdB1 gene, and a 1.2-kb EcoRV fragment containing the pIK03-derived kanamycin resistance gene was inserted therein (the underlined portion of the sequence of SEQ ID NO: 1). Insertion of the fragment in the same direction as the pBluescript II KS(+) lac promoter transcription direction was confirmed by SmaI digestion, thus obtaining pKS78F. A 4.9-kb SalI-EcoRV fragment was cut out from pKS78F and inserted at the SalI-SmaI site of pK19 mobsacB to construct plasmid pDBKM for generated of a pmdB1-disrupted strain (Fig. 4).

### (1) Preparation of pmdB1 gene-disrupting plasmid

### (2) Construction of pmdB1 gene-disrupted strain

E6 precultured in 3 ml of LB medium was inoculated at 1% into 10 ml of LB medium, for main culturing. After growing at OD600=0.5, the culture was centrifuged at 5,000 rpm, 4°C, 15 minutes and the cells were collected. After rinsing twice in 1 ml of 0.3 M sucrose, it was suspended in 1 ml of 0.5 M sucrose. Next, 1 µl of the gene-disrupting plasmid pDBKM prepared to 1 µg/µl was added to 100 µl of the culture and subjected to pulsing using a Gene pulser (Bio-Rad), under conditions with a resistance of 800 Q, a voltage of 12 V and an electrostatic capacity of 25 µF. Immediately after pulsing, 1 ml of LB medium was added and culturing was carried out at 30°C for 6 hours. A 300 µl portion thereof was coated onto 100 µg/ml kanamycin-containing LB medium.
The obtained kanamycin-resistant strains were collected with 1 ml of LB medium, seeded in 10 ml of LB medium containing 10% sucrose, and cultured for 12 hours. A 200 µl portion was then transferred into fresh LB medium and the procedure was repeated three times. Finally, the culture solution was coated onto 100 µg/ml kanamycin-containing LB medium and the obtained colonies were used as candidate gene-disrupted strains.

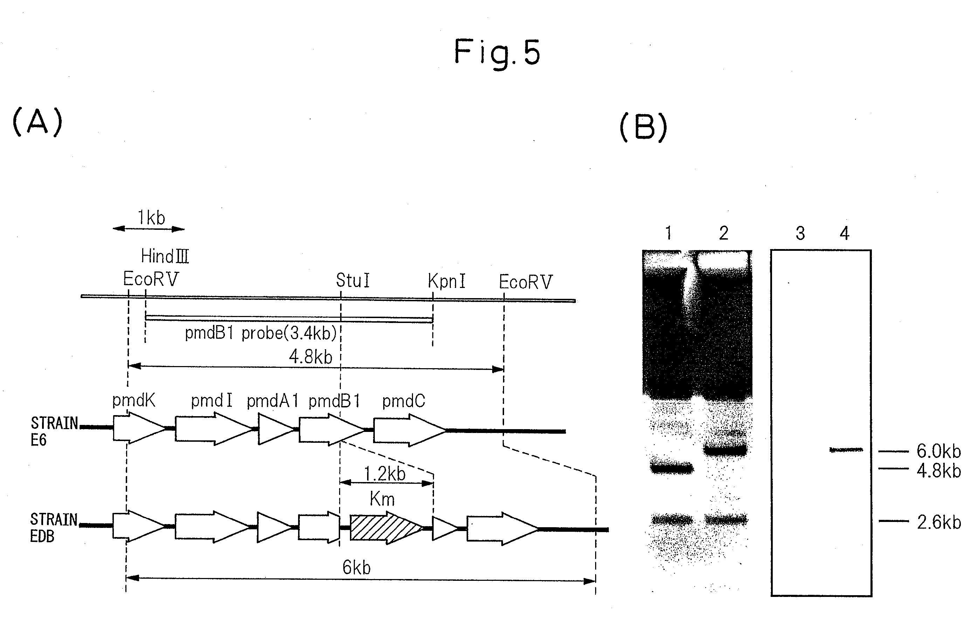

The candidate gene-disrupted strains were precultured in 3 ml of 100 µg/ml kanamycin-containing LB medium, and inoculated at 1% into 10 ml of culture medium for main culturing. Next, the total DNA of the candidate gene-disrupted strains were recovered and digested with EcoRV, after which a 3.4-kb HindIII-KpnI fragment containing pmdA1B1 and a 1.2-kb EcoRV fragment containing the pIK03-derived kanamycin resistance gene were used as probes for Southern hybridization (Fig. 5). Lanes 1 and 3 represent the total DNA of the wild type E6 digested with EcoRV, and lanes 2 and 4 represent the total DNA of the gene-disrupted strain digested with EcoRV. The probe used was a pmdB1-containing 3,4-kb HindIII-KpnI fragment for lanes 1 and 2 and a kanamycin (Km) resistance gene-containing 1.2-kb EcoRV fragment for lanes 3 and 4.

### (3) Confirmation of pmdB1 gene disruption

### Example 3: Production of 3-carboxymuconolactone

### (1) Construction of recombinant plasmid pKTphHG/C for 3-carboxy-cis,cis-muconic acid

1-2) Next, a DNA fragment obtained by cutting chloramphenicol resistance gene-containing plasmid pHSG398 (product of Takara) with restriction enzyme Cfr13I and then blunting the ends, and a DNA fragment obtained by cutting pKHG with restriction enzyme KpnI and then blunting the ends, were ligated with T4DNA ligase (Roche) to construct recombinant plasmid pKHG/C (Fig. 7).

1-3) Also, a DNA fragment obtained by cutting recombinant plasmid pHE96/pBluescript II SK(+) mentioned in Japanese Patent Application No. 2006-218524 with restriction enzyme EcoRI and a DNA fragment obtained by cutting pKHG/C with restriction enzyme EcoRI were ligated with T4DNA ligase (Roche) to construct recombinant plasmid pKTphHG/C (Fig. 8).

### (2) Transformation

2-1) Recombinant plasmid pKTphHG/C was used to transform *E. coli* HB101, and the transformants were shake cultured at 37°C for 18 hours in LB medium (100 ml) containing 25 mg/L kanamycin, after which the recombinant plasmid pKTphHG/C was extracted from the proliferated cultured cells.

2-2) The gene-disrupted strain generated in Example 2 (Comamonas sp. EDB) was cultured at 28°C for 23 hours in 500 ml of LB liquid medium and cooled on ice for 30 minutes. The cells were collected by centrifugation at 4°C, 10,000 rpm for 10 minutes, and after mild rinsing with 500 ml of 0°C distilled water, they were recentrifuged. This was followed by additional mild rinsing with 250 ml of 0°C distilled water and re-centrifugation. This was further followed by additional mild rinsing with 125 ml of 0°C distilled water and re-centrifugation. The collected microbial cells were suspended in distilled water containing 10% glycerol and stored at 0°C.

2-3) After placing 4 µl of distilled water containing about 0.05 µg of the DNA of plasmid pKTphHG/C of (1) in a 0.2 cm cuvette, 40 µl of the cell solution suspended in distilled water containing 10% glycerol obtained in 2-2) above was added, and the mixture was subjected to electroporation under conditions of 25 µF, 2500 V, 12 msec.

2-4) The total amount of treated cells was seeded in 10 ml of LB liquid medium and cultured at 28°C for 6 hours. After culturing, the cells were collected by centrifugation, developed on an LB plate containing 25 mg/L kanamycin, 50 mg/L ampicillin and 30 mg/L chloramphenicol and cultured at 28°C for 48 hours, to obtain transformants retaining plasmid pKTphHG/C and exhibiting chloramphenicol resistance. The cells were designated as strain pKTphHG/C/Comamonas sp. ECB.

2-5) Strain pKTphHG/C/Comamonas sp. ECB was seeded in 200 ml of LB liquid medium (containing 30 mg/L chloramphenicol) and cultured at 28°C for 16 hours, to produce a cultured cell suspension. After preparing 5 L of LB liquid medium and 3 ml of antifoaming agent (Antifoam A) using a 10 L-volume jar fermenter, an aqueous solution obtained by dissolving 200 ml of a precultured cell suspension of pKTphHG/C/Comamonas sp. ECB cultured therein and 8 g of terephthalic acid was mixed therewith, and aerated stirring was carried out at 500 rpm/min at 28°C, for culturing to OD518 = approximately 3 (8 hours-12 hours).

2-6) When the OD518 reached 3 with culturing using a 10 L-volume jar fermenter, 500 ml of culture solution was removed from the fermenter into an Erlenmeyer flask and stored on ice.

2-7) To the culture solution in the fermenter that had reached OD518 of 3 there was added 42 g of terephthalic acid dissolved in 500 ml of a 0.1 N NaOH aqueous solution (adjusted to pH 8.5), over a period of 10-12 hours using a peristaltic pump. In order to prevent reduction in the pH of the culture solution with production of 3-carboxy-cis,cis-muconic acid as the reaction proceeded, a 0.1 N NaOH solution was added with a peristaltic pump connected to a pH sensor to maintain the pH of the culture solution. Progress of the reaction was confirmed by HPLC. After 36 hours, the added terephthalic acid had virtually disappeared. A 500 ml portion of the ice-cooled cell suspension prepared in 2-5) was added to the culture solution in the fermenter and culturing was continued for 12 hours.

2-8) Upon completion of the reaction, the medium in the fermenter was transferred to a plastic container (bucket). The cell component was precipitated and removed from the culture solution by centrifugal separation (6000 rpm, 20°C), hydrochloric acid was added to the obtained supernatant to lower the pH to below 1.0, and the mixture was stored at low temperature for conversion of the 3-carboxy-cis,cis-muconic acid to 3-carboxymuconolactone. After confirming complete conversion to 3-carboxymuconolactone by GC-MS, an organic solvent (ethyl acetate) was used for extraction of the 3-carboxymuconolactone. The amount of extracted and dried 3-carboxymuconolactone reached approximately 9.8 g from 1 L of culture solution, which was a yield of about 87% as the ratio of added substrate (terephthalic acid). The obtained 3-carboxymuconolactone was further treated with active carbon and the structure was confirmed by its NMR and MS spectra.
¹H-NMR (400 MHz, DMSOd₆)δ: 2.67, 3.10, 5.55, 6.81, 12.5-13.0
¹³C-NMR (100 MHz, DMSOd₆)δ: 36.5, 78.5, 125.9, 157.9, 162.1, 170.4, 170.8
MS m/z: 330 (M⁺) (as TMS (trimethylsilyl) form of 3-carboxymuconolactone)

## Claims

1. A protocatechuate 4,5-ring-cleaving enzyme gene-disrupted strain in which the gene coding for:
(a) the amino acid sequence set forth in SEQ ID NO: 1 or 3, or
(b) the amino acid sequence set forth in SEQ ID NO: 1 or 3 which has a deletion, substitution, addition and/or insertion of one or more amino acids and exhibits protocatechuate 4,5-ring cleavage activity,
present in the chromosomal DNA of microbial cells, has been disrupted.

2. A protocatechuate 4,5-ring-cleaving enzyme gene-disrupted strain in which a gene that:
(a) comprises the nucleotide sequence set forth in SEQ ID NO: 2 or 4; or
(b) is a nucleotide sequence hybridizing with DNA consisting of a nucleotide sequence complementary to the nucleotide sequence of (a), under stringent conditions, and coding for an enzyme with protocatechuate 4,5-ring cleavage activity,
present in the chromosomal DNA of microbial cells, has been disrupted.

3. A gene-disrupted strain according to claim 1 or 2, in which the protocatechuate 4,5-ring-cleaving enzyme gene has been disrupted by homologous recombination between a gene coding for:
(a) the amino acid sequence set forth in SEQ ID NO: 1 or 3, or
(b) the amino acid sequence set forth in SEQ ID NO: 1 or 3 which has a deletion, substitution, addition and/or insertion of one or more amino acids and exhibits protocatechuate 4,5-ring cleavage activity,
present in the chromosomal DNA of microbial cells, and homologous recombination DNA having a DNA sequence that can undergo homologous recombination with the gene and lacking protocatechuate 4,5-ring cleavage activity.

4. A gene-disrupted strain according to any one of claims 1 to 3, wherein the parent strain of the protocatechuate 4,5-ring-cleaving enzyme gene-disrupted strain is a Comamonas sp. bacterium.

5. A gene-disrupted strain according to claim 4, wherein the Comamonas sp. bacterium is Comamonas sp. E6.

6. A recombinant plasmid comprising a terephthalate dioxygenase gene (TPA-DOX gene), NADPH-reductase gene, 1,2-dihydroxy-3,5-cyclohexadiene-1,4-dicarboxylate dehydrogenase gene (DCD dehydrogenase gene), positive regulator gene, terephthalate transporter gene (TPA transporter gene) and protocatechuate 3,4-dioxygenase gene (pcaHG gene).

7. A transformant obtained by introducing a recombinant plasmid according to claim 6 into a gene-disrupted strain according to any one of claims 1 to 5.

8. A process for production of 3-carboxy-cis,cis-muconic acid and/or 3-carboxymuconolactone, **characterized by** culturing a transformant according to claim 7 in the presence of terephthalic acid.
